(19) Europäisches Patentamt / European Patent Office / Office européen des brevets

(11) **EP 4 241 768 A1**

(12) **EUROPEAN PATENT APPLICATION**
published in accordance with Art. 153(4) EPC

(43) Date of publication:
**13.09.2023 Bulletin 2023/37**

(21) Application number: **21889154.7**

(22) Date of filing: **01.11.2021**

(51) International Patent Classification (IPC):
**A61K 31/015** (2006.01)      **A23L 33/10** (2016.01)
**A61K 8/31** (2006.01)        **A61P 9/10** (2006.01)
**A61P 9/12** (2006.01)        **A61P 9/14** (2006.01)
**A61Q 19/00** (2006.01)

(52) Cooperative Patent Classification (CPC):
**A23L 33/10; A61K 8/31; A61K 31/015; A61P 9/10;
A61P 9/12; A61P 9/14; A61Q 19/00**

(86) International application number:
**PCT/JP2021/040234**

(87) International publication number:
**WO 2022/097601 (12.05.2022 Gazette 2022/19)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **06.11.2020 US 202063110506 P**

(71) Applicants:
• **Kinki University
Higashi-Osaka-shi
Osaka 577-8502 (JP)**
• **Inabata Koryo Co., Ltd.
Osaka-shi, Osaka 532-0027 (JP)**
• **Sunsho Pharmaceutical Co., Ltd.
Fuji-shi, Shizuoka 419-0201 (JP)**

(72) Inventors:
• **ZAIMA Nobuhiro
Higashiosaka-shi, Osaka 577-8502 (JP)**

• **KISHI Chihiro
Higashiosaka-shi, Osaka 577-8502 (JP)**
• **TAKEMOTO Yuki
Higashiosaka-shi, Osaka 577-8502 (JP)**
• **MATSUMURA Shinichi
Osaka-shi, Osaka 532-0027 (JP)**
• **YOSHIOKA Yuri
Osaka-shi, Osaka 532-0027 (JP)**
• **IWAMOTO Kohei
Osaka-shi, Osaka 532-0027 (JP)**
• **MAKINO Shohei
Osaka-shi, Osaka 532-0027 (JP)**
• **YAMADA Kazuya
Fuji-shi, Shizuoka 419-0201 (JP)**
• **KOBAYASHI Takanori
Fuji-shi, Shizuoka 419-0201 (JP)**

(74) Representative: **Kinkeldey, Daniela
Bird & Bird LLP
Maximiliansplatz 22
80333 München (DE)**

(54) **COMPOSITION FOR TREATING VASCULAR DISEASE, COMPOSITION FOR PREVENTING VASCULAR DISEASE, COMPOSITION FOR TREATING HYPERTENSION, AND COMPOSITION FOR PREVENTING HYPERTENSION**

(57)    Provided is a composition for the treatment of vascular disease, for the prevention of vascular disease, for the treatment of hypertension or for the prevention of hypertension, comprising caryophyllene.

EP 4 241 768 A1

[Figure 2]

**Description**

[Technical Field]

The present invention relates to a composition for the treatment of vascular disease, a composition for the prevention of vascular disease, a composition for the treatment of hypertension, and a composition for the prevention of hypertension. More specifically, the present invention relates to a composition for the treatment of vascular disease, a composition for the prevention of vascular disease, a composition for the treatment of hypertension, and a composition for the prevention of hypertension, comprising caryophyllene

[Background Art]

"Rupture and dissection of aortic aneurysm" as vascular disease, a disease site of which is a vascular wall, are the ninth leading cause of death of Japanese. Nonetheless, any effective drug therapy or prevention method other than surgery has not yet been established. Examples of the risk factor of the disease include age, sex, hypertension, and arteriosclerosis. Particularly, the influence of smoking is pointed out. A putative mechanism under which smoking causes abdominal aortic aneurysm is known to involve nicotine which activates G protein-coupled receptor (GPCR) on cell surface and induces oxidative stress and cytokine synthesis, followed by intracellular signal transduction and the induction of matrix metalloprotease (MMP) synthesis, thereby promoting the degradation of extracellular matrix in arterial vascular elastic lamina (Non Patent Literature 1). The causes of abdominal aortic aneurysm, other than nicotine uptake, ascribable to smoking are considered to also start at oxidative stress and cytokine synthesis and undergo a common mechanism. Thus, for preventing the progression of abdominal aortic aneurysm, it is important to suppress the degradation of vascular extracellular matrix starting at oxidative stress and cytokine synthesis. Fish oil, sesame extracts, isoflavone, black soybean extracts, and the like, which have an antioxidative effect and also have high bioavailability, have heretofore been known as foods that prevent the degradation of vascular extracellular matrix (Non Patent Literatures 2, 3, 4 and 5). There is also a report stating that green tea-derived polyphenol prevents abdominal aortic aneurysm (Non Patent Literature 6).
Some foods having an antioxidative effect do not have an effect of preventing the degradation of vascular extracellular matrix. Thus, the possession of an antioxidative effect does not always mean the possession of an effect of preventing the degradation of vascular extracellular matrix.
Furthermore, a problem of the prevention of the degradation of vascular extracellular matrix using fish oil, sesame extracts, isoflavone, black soybean extracts, green tea-derived polyphenol, and the like is the uptake of such a component in a large amount in foods. As an example, sesame extracts require the daily uptake of approximately 10 mg per day, leading to a great deal of time and labor and financial burden.

[Citation List]

[Non Patent Literature]

[Non Patent Literature 1] Shuangxi Wang, Cheng Zhang, Miao Zhang, Bin Liang, Huaiping Zhu, Jiyeon Lee, Benoit Viollet, Lijun Xia, Yun Zhang, Ming-Hui Zou, Nature Medicine, 2012, 18 (6), 902-912.
[Non Patent Literature 2] Hirona Kugo, Nobuhiro Zaima, Megumi Onozato, Chie Miyamoto, Keisuke Hashimoto, Kenichi Yanagimoto, Tatsuya Moriyama, Food Funct., 2017, 8, 2829-2835.
[Non Patent Literature 3] Hirona Kugo, Chie Miyamoto, Ayaka Sawaragi, Kiyoto Hoshino, Yuka Hamatani, Shinichi Matsumura, Yuri Yoshioka, Tatsuya Moriyama, Nobuhiro Zaima, J. Oleo Sci., 2019, 68 (1), 79-85.
[Non Patent Literature 4] Wanida Sukketsiri, Kiyoto Hoshino, Hirona Kugo, Tomomi Nakamura, Tsukasa Sasoh, Tatsuya Moriyama, Nobuhiro Zaima, J. Oleo Sci., 2019, 68, 1241-1249.
[Non Patent Literature 5] Kiyoto Hoshino, Hirona Kugo, Chie Miyamoto, Keisuke Hashimoto, Hiroshi Murase, Masatoshi Mizuno, Tatsuya Moriyama, Nobuhiro Zaima, J. Nutr. Sci. Vitaminol., 2020, 66, 75-81.
[Non Patent Literature 6] Shuji Setozaki, Kenji Minakata, Hidetoshi Masumoto, Shingo Hirao, Kazuhiro Yamazaki, Koichiro Kuwahara, Tadashi Ikeda, Ryuzo Sakata, J. Vascular Surgery, 2017, 65, 180-1812.

[Summary of Invention]

[Technical Problem]

Under these circumstances, there is a demand for a composition that can readily treat or prevent vascular disease or hypertension and a method that can treat or prevent vascular disease or hypertension.

[Solution to Problem]

The present invention includes the following aspects.

[1] A composition for the treatment of vascular disease, for the prevention of vascular disease, for the treatment of hypertension or for the prevention of hypertension, comprising caryophyllene.
[2] The composition according to [1], wherein the caryophyllene improves arterial stiffness or suppresses increase in arterial stiffness.
[3] The composition according to [1], wherein the caryophyllene suppresses the degradation of vascular extracellular matrix.
[4] The composition according to [1], wherein the caryophyllene suppresses the destruction of vascular fibra or repairs vascular fibra.
[5] The composition according to [1], wherein the caryophyllene suppresses the destruction of vascular elastic lamina or repairs vascular elastic lamina.
[6] The composition according to [1], wherein the caryophyllene inhibits matrix metalloprotease.
[7] The composition according to [1], wherein the caryophyllene suppresses blood pressure elevation.
[8] A composition for improving skin quality, comprising caryophyllene.
[9] The composition according to any of [1] to [8], wherein the caryophyllene is obtained from clove, caraway, basil, oregano, hop, cinnamon, Ceylon cinnamon tree, rosemary, *Cannabis L.,* hemp, cannabis, black pepper, lavender, malabathrum, cananga tree, copaiba, melegueta pepper, curry leaf and/or an essential oil thereof.
[10] The composition according to any of [1] to [9], wherein the caryophyllene is a chemically synthesized compound.
[11] The composition according to any of [1] to [10], further comprising one or more members selected from the group consisting of a solvent and a fragrance.
[12] The composition according to any of [1] to [11], wherein a dosage form is a capsule.
[13] A tobacco filter comprising the composition according to any of [1] to [11].
[14] A tobacco filter incorporating the capsule according to [12].
[15] A tobacco comprising the tobacco filter according to [13] or [14].
[16] A smoking tool comprising the tobacco filter according to [13] or [14].
[17] A tobacco comprising the composition according to any of [1] to [11].
[18] A food or drink comprising the composition according to any of [1] to [12].
[19] A medicament comprising the composition according to any of [1] to [12].
[20] The medicament according to [19], wherein the medicament is an oral composition or a pulmonary composition.
[21] A method for treating vascular disease, a method for treating hypertension, a method for preventing vascular disease, or a method for preventing hypertension, comprising administering a composition comprising caryophyllene.

[21-1] The method according to [21], wherein the caryophyllene is administered to a patient who has taken nicotine.
[21-2] The method according to [21], wherein the caryophyllene is administered with nicotine.
[21-3] The method according to [21], wherein the vascular disease or the hypertension is a disease induced by the uptake of nicotine.

[22] The method according to [21], wherein the caryophyllene improves arterial stiffness or suppresses increase in arterial stiffness to treat or prevent vascular disease or hypertension.
[23] The method according to [21], wherein the caryophyllene suppresses the degradation of vascular extracellular matrix to treat or prevent vascular disease or hypertension.
[24] The method according to [21], wherein the caryophyllene suppresses the destruction of vascular fibra or repairs vascular fibra to treat or prevent vascular disease or hypertension.
[25] The method according to [21], wherein the caryophyllene suppresses the destruction of vascular elastic lamina or repairs vascular elastic lamina to treat or prevent vascular disease or hypertension.
[26] The method according to [21], wherein the caryophyllene inhibits matrix metalloprotease to treat or prevent vascular disease or hypertension.

[27] The method according to [21], wherein the caryophyllene suppresses blood pressure elevation to treat or prevent vascular disease or hypertension.

[28] A method for improving skin quality, comprising administering a composition comprising caryophyllene.

[29] The method according to any of [21] to [28], wherein the administration is pulmonary uptake or oral uptake.

[30] The method according to [29], wherein the pulmonary uptake is inhalation mediated by a filter of an inhalation tool.

[31] A composition comprising caryophyllene for use in the treatment of vascular disease, the treatment of hypertension, the prevention of vascular disease, or the prevention of hypertension.

[31-1] The composition according to [31], wherein the caryophyllene is administered to a patient who has taken nicotine.

[31-2] The composition according to [31], wherein the caryophyllene is administered with nicotine.

[31-3] The composition according to [31], wherein the vascular disease or the hypertension is a disease induced by the uptake of nicotine.

[32] The composition according to [31], wherein the caryophyllene improves arterial stiffness or suppresses increase in arterial stiffness to perform the treatment of vascular disease, the treatment of hypertension, the prevention of vascular disease, or the prevention of hypertension.

[33] The composition according to [31], wherein the caryophyllene suppresses the degradation of vascular extracellular matrix to perform the treatment of vascular disease, the treatment of hypertension, the prevention of vascular disease, or the prevention of hypertension.

[34] The composition according to [31], wherein the caryophyllene suppresses the destruction of vascular fibra to perform the treatment of vascular disease, the treatment of hypertension, the prevention of vascular disease, or the prevention of hypertension.

[35] The composition according to [31], wherein the caryophyllene suppresses the destruction of vascular elastic lamina to perform the treatment of vascular disease, the treatment of hypertension, the prevention of vascular disease, or the prevention of hypertension.

[36] The composition according to [31], wherein the caryophyllene inhibits matrix metalloprotease to perform the treatment of vascular disease, the treatment of hypertension, the prevention of vascular disease, or the prevention of hypertension.

[37] The composition according to [31], wherein the caryophyllene suppresses blood pressure elevation to perform the treatment of vascular disease, the treatment of hypertension, the prevention of vascular disease, or the prevention of hypertension.

[38] A composition comprising caryophyllene for use in the improvement of skin quality.

In the present specification, the term "%" refers to percent by mass unless otherwise specified.

[Advantageous Effects of Invention]

A preferred aspect of the present invention can provide a composition that can readily treat or prevent vascular disease or hypertension and a method that can treat or prevent vascular disease or hypertension.

[Brief Description of Drawings]

[Figure 1] Figure 1 shows a caryophyllene-filled flask used in Examples.

[Figure 2] Figure 2 shows change in the serum concentration of $\beta$-caryophyllene estimated in smoking 20 tobaccos a day at a frequency of one tobacco per hour by disintegrating a capsule within a filter comprising an easy-to-disintegrate seamless capsule supplemented with 15% by mass of $\beta$-caryophyllene.

[Description of Embodiments]

[Caryophyllene]

The composition according to one aspect of the present invention comprises caryophyllene.

Examples of the caryophyllene include $\beta$-caryophyllene, $\alpha$-caryophyllene, isocaryophyllene, and metabolites or derivatives of caryophyllene (e.g., caryophyllene oxide such as $\beta$-caryophyllene oxide). The composition according to one aspect of the present invention may comprise these components singly or in combinations of two or more thereof.

Usually, the caryophyllene preferably comprises β-caryophyllene and may further comprise caryophyllene other than β-caryophyllene [e.g., at least one member selected from α-caryophyllene, isocaryophyllene, and metabolites or derivatives of caryophyllene].

In such a composition comprising β-caryophyllene, the ratio of the β-caryophyllene to the whole caryophyllene may be, for example, 30% by mass or more, 50% by mass or more, 70% by mass or more, 80% by mass or more, 90% by mass or more, 95% by mass or more, or 100% by mass (substantially 100% by mass).

In the present specification, the term "β-caryophyllene" may be used as a generic name including such caryophyllene other than β-caryophyllene.

The caryophyllene such as β-caryophyllene may be derived from, for example, but not particularly limited to, clove, caraway, basil, oregano, hop, cinnamon, Ceylon cinnamon tree, rosemary, *Cannabis L.,* hemp, cannabis, black pepper, lavender, malabathrum, cananga tree, copaiba, melegueta pepper, curry leaf, and essential oil thereof(e.g., which may be extracts or concentrates).

The caryophyllene to be used may be a commercially available product or may be produced (purified) (chemically synthesized) by a routine method.

The content of the caryophyllene in the composition according to one aspect of the present invention is not particularly limited and can be appropriately selected according to a desired function (e.g., the promotion of volatilization, the promotion of dissolution, and freezing resistance as well as the function of caryophyllene), dosage form, etc. The content (ratio or concentration) of the caryophyllene in the composition according to one aspect of the present invention may be, for example, 0.01% by mass or more, 0.05% by mass or more, 0.1% by mass or more, 0.5% by mass or more, 1% by mass or more, 5% by mass or more, 10% by mass or more, 15% by mass or more, 20% by mass or more, 25% by mass or more, 30% by mass or more, 35% by mass or more, 40% by mass or more, 45% by mass or more, 50% by mass or more, 55% by mass or more, 60% by mass or more, 65% by mass or more, 70% by mass or more, 75% by mass or more, 80% by mass or more, 85% by mass or more, 90% by mass or more, or 95% by mass or more, and may be, for example, 99.9% by mass or less, 99.5% by mass or less, 99% by mass or less, 95% by mass or less, 90% by mass or less, 80% by mass or less, 70% by mass or less, 60% by mass or less, 50% by mass or less, 40% by mass or less, or 30% by mass or less.

The concentration (ratio) range of the caryophyllene may be set by appropriately combining the lower limit value and the upper limit value of the range (e.g., 0.1 to 90% by mass and 10 to 50% by mass) (the same holds true for all the ranges described in the present specification). Thus, examples of the content of the caryophyllene in the composition according to one aspect of the present invention include 1% by mass or more, 3 to 99% by mass, 5 to 80% by mass, 5 to 90% by mass, and 15 to 30% by mass.

Likewise, when the composition comprises an oil/fat (or the caryophyllene is combined with an oil/fat), the ratio (concentration) of the caryophyllene to the total amount [100% by mass in total] of the caryophyllene and the oil/fat may also be selected from the concentration range of the caryophyllene (e.g., 1% by mass or more, 10% by mass or more, and 5 to 90% by mass) in the composition described above.

The caryophyllene is known (reported) to have functions such as the relief (improvement or suppression) of anxiety [e.g., motion sickness, nocturnal enuresis, and stress hives], the relief (improvement or suppression) of stress, the inhibition of β-secretase (inhibition of β-secretase activity), and the prevention or improvement of major neurocognitive disorder (or dementia, e.g., senile dementia such as major neurocognitive disorder due to Alzheimer's disease (Alzheimer-type dementia)). Hence, the composition of the present invention may be used for the purpose (in the application) of conferring (or obtaining) such a function (effect), though depending on its form of usage.

The intake of the caryophyllene per day is not particularly limited and may be, for example, 0.1 mg or more, 0.5 mg or more, 1.0 mg or more, 2.0 mg or more, 3.0 mg or more, 4.0 mg or more, 5.0 mg or more, 6.0 mg or more, 7.0 mg or more, 10.0 mg or more, 15.0 mg or more, 20.0 mg or more, 30.0 mg or more, 40.0 mg or more, 50.0 mg or more, 60.0 mg or more, 70.0 mg or more, 80.0 mg or more, 90.0 mg or more, 100.0 mg or more, 150.0 mg or more, 200.0 mg or more, 250.0 mg or more, 300.0 mg or more, 350.0 mg or more, 400.0 mg or more, 450.0 mg or more, 500.0 mg or more, 550.0 mg or more, 600.0 mg or more, 650.0 mg or more, 700.0 mg or more, 750.0 mg or more, 800.0 mg or more, 850.0 mg or more, 900.0 mg or more, 950.0 mg or more, 1000.0 mg or more, 1100.0 mg or more, 1200.0 mg or more, 1300.0 mg or more, 1400.0 mg or more, 1500.0 mg or more, 1600.0 mg or more, 1700.0 mg or more, 1800.0 mg or more, 1900.0 mg or more, or 2000.0 mg or more, and may be 5000.0 mg or less, 4500.0 mg or less, 4000.0 mg or less, 3500 mg or less, 3000.0 mg or less, 2500.0 mg or less, 2000.0 mg or less, 1900.0 mg or less, 1800.0 mg or less, 1700.0 mg or less, 1600.0 mg or less, 1500.0 mg or less, 1400.0 mg or less, 1300.0 mg or less, 1200.0 mg or less, 1100.0 mg or less, 1000.0 mg or less, 950.0 mg or less, 900.0 mg or less, 850.0 mg or less, 800.0 mg or less, 750.0 mg or less, 700.0 mg or less, 650.0 mg or less, 600.0 mg or less, 550.0 mg or less, 500.0 mg or less, 450.0 mg or less, 400.0 mg or less, 350.0 mg or less, 300.0 mg or less, 250.0 mg or less, 200.0 mg or less, 150.0 mg or less, 100.0 mg or less, 90.0 mg or less, 80.0 mg or less, 70.0 mg or less, 60.0 mg or less, 50.0 mg or less, 40.0 mg or less, 30.0 mg or less, 20.0 mg or less, 15.0 mg or less, 14.0 mg or less, 13.0 mg or less, 12.0 mg or less, 11.0 mg or less, 10.0 mg or less, 9.0 mg or less, 8.0 mg or less, 7.0 mg or less, 6.0 mg or less, 5.0 mg or less, 4.0 mg or less, 3.0 mg or less, 2.0 mg or less, 1.0 mg or less, 0.9 mg or less, 0.8 mg or less, 0.7 mg or less, 0.6 mg or less, 0.5 mg or less, 0.4 mg or less, 0.3 mg or less, 0.2 mg or less, or 0.1 mg or less.

[Composition]

The composition according to one aspect of the present invention may comprise other components according to its form, application, recipient, etc. Examples thereof include oils/fats, carriers, excipients, binders, disintegrants, lubricants, coating agents, colorants, stabilizers, emulsifiers (surfactants), absorption promoters, gelling agents, pH adjusters, antiseptics, antioxidants, refrigerants, physiologically active substances, bioactive substances, microbes, foods and drinks, plants, sweeteners, acidulants, flavorings, tonics, and fragrances. These components may be used singly or in combinations of two or more thereof.

Examples of the oil/fat include plant oils (e.g., soybean oil, rapeseed oil, corn oil, sesame oil, flax seed oil, cotton seed oil, perilla oil, olive oil, rice oil, palm oil, jojoba oil, sunflower seed oil, and camellia oil), animal oils (e.g., beef tallow, lard, chicken fat, cream, fish oil, and horse oil), and medium-chain triglyceride (MCT).

These oils/fats may be used singly or in combinations of two or more thereof.

Among these oils/fats, particularly, MCT may be suitably used. Hence, the oil/fat may comprise at least MCT. When the oil/fat thus comprises MCT, the ratio of the MCT to the whole oil/fat may be, for example, 10% by mass or more, 30% by mass or more, 50% by mass or more, 70% by mass or more, 80% by mass or more, 90% by mass or more, or 100% by mass (only MCT).

The oil/fat functions as a vehicle (solvent or carrier) in the composition. In addition, depending on its type (e.g., MCT), the volatility or solubility of a fragrance, the freezing resistance of a fragrance or the oil/fat itself, and the like are relatively favorable (or not largely reduced) in many cases. From such a viewpoint, a portion of the caryophyllene, etc. may be suitably replaced with the oil/fat (particularly, MCT, etc.). Also, use of the oil/fat (MCT, etc.) may be advantageous in encapsulation or the like. Furthermore, the oil/fat seems to rarely influence aroma and is easily used in combination with a fragrance or the caryophyllene.

The ratio (amount or concentration) of the oil/fat in the composition according to one aspect of the present invention may be, for example, 0.1% by mass or more (e.g., 0.5% by mass or more), 1% by mass or more (e.g., 5% by mass or more), 10% by mass or more (e.g., 15% by mass or more), 20% by mass or more (e.g., 25% by mass or more), 30% by mass or more (e.g., 35% by mass or more), 40% by mass or more (e.g., 45% by mass or more), 50% by mass or more (e.g., 55% by mass or more), 60% by mass or more (e.g., 65% by mass or more), 70% by mass or more (e.g., 75% by mass or more), 80% by mass or more (e.g., 85% by mass or more), or 90% by mass or more (e.g., 95% by mass or more), and may be, for example, 99% by mass or less (e.g., 95% by mass or less), 90% by mass or less, 80% by mass or less, 70% by mass or less, 60% by mass or less, 50% by mass or less, 40% by mass or less, 30% by mass or less, or 20% by mass or less.

Examples of the carrier that may be contained in the composition according to one aspect of the present invention include, but are not particularly limited to, acids (e.g., fatty acids such as caprylic acid, capric acid, eicosapentaenoic acid, docosahexaenoic acid, oleic acid, and linoleic acid), hydrocarbons (e.g., liquid paraffin, squalane, and Vaseline), silicones (e.g., silicone oil), synthetic polymers (e.g., polyacrylic acid, carboxyvinyl polymers, polyethylene glycol, and polyvinylpyrrolidone), natural polymers or their derivatives (e.g., carrageenan, alginic acid, cellulose, guar gum, xanthan gum, quince seeds, dextran, gellan gum, hyaluronic acid, ethylcellulose, hydroxypropylcellulose, hydroxypropylmethylcellulose, cationized guar gum, acetylated hyaluronic acid, and sodium alginate), lower alcohols (e.g., ethanol and isopropanol), polyhydric alcohols (e.g., ethylene glycol, glycerin, propylene glycol, butylene glycol, diglycerin, and dipropylene glycol), and water.

The nature of the carrier can be selected according to the form of the composition, etc., and the carrier may be solid or liquid, for example, and may be non-volatile or volatile. The liquid carrier may be regarded as a solvent.

Examples of the emulsifier (surfactant) that may be contained in the composition according to one aspect of the present invention include, but are not particularly limited to, nonionic surfactants [e.g., sugar fatty acid ester (e.g., sucrose fatty acid ester, maltose fatty acid ester, and lactose fatty acid ester), propylene glycol fatty acid ester, glycerin fatty acid ester, sorbitan fatty acid ester, polyglycerin fatty acid ester, and organic acid monoglyceride].

In the case of using an emulsifier, the ratio (concentration) of the emulsifier in the composition can be selected according to the form of the composition, application, etc. and is not particularly limited. The ratio can be selected from, for example, the range of approximately 40% by mass or less (e.g., 35% by mass or less), and may be, for example, 30% by mass or less (e.g., 25% by mass or less), preferably 20% by mass or less (e.g., 15% by mass or less), more preferably 10% by mass or less (e.g., 8% by mass or less) or 5% by mass or less (e.g., 4% by mass or less, 3% by mass or less, and 1% by mass or less).

The lower limit value of the ratio (concentration) of the emulsifier can be selected according to the form of the composition, application, etc. and is not particularly limited. The lower limit value may be, for example, 0.01% by mass, 0.1% by mass, 0.5% by mass, 0.7% by mass, 1% by mass, 1.2% by mass, 1.5% by mass, 2% by mass, or 3% by mass.

The emulsifier is capable of largely reducing interfacial surface tension in the composition. Hence, when the composition is used as contents of a capsule (e.g., a seamless capsule produced by a dropping method or the like), encapsulation is easily inhibited. From such a viewpoint, in the case of using the composition as contents of a capsule, it is desirable that the emulsifier should not be used (should not be substantially used). It is desirable that the ratio of the emulsifier, if used, should be relatively small (e.g., 5% by mass or less or 3% by mass or less of the composition).

The composition according to one aspect of the present invention may comprise at least one component selected from dicarboxylic acid ester, diol ester, monocarboxylic acid ester, ester of polyol having three or more hydroxy groups, ester of polycarboxylic acid having three or more carboxy groups, polyol ether, polyamine, and an alcohol having 6 or more carbon atoms.

Such a component is capable of functioning as a vehicle (solvent or carrier). In addition, the solubility of a fragrance (e.g., a menthol-containing fragrance), the freezing resistance of a fragrance or the oil/fat itself, and the like are relatively favorable in many cases. Particularly, such a component may be capable of improving the solubility of a fragrance or the freezing resistance of a fragrance or the oil/fat itself by combination with the caryophyllene.

The administration of the composition according to one aspect of the present invention includes a route selected from the group consisting of oral, parenteral, pulmonary, intraperitoneal, intravenous, intraarterial, transdermal, sublingual, intramuscular, intrarectal, transbuccal, transnasal, inhalation-mediated, intravaginal, intraocular, local delivery-mediated, subcutaneous, intra-fat, intra-articular, and intrathecal routes, and may be performed through a wide range of nonlimiting administration routes. In one specific variation, the administration is performed orally or pulmonarily.

The oral administration may involve swallowing when the composition enters blood flow via the digestive tract. Alternatively or additionally, the oral administration may involve mucosal administration (e.g., buccal, sublingual or lingual administration) when the compound enters blood flow via the mucous membrane of mouth.

Examples of the preparation suitable for oral administration include solid, semisolid, and liquid systems, for example, tablets; soft or hard capsules containing multiparticles or nanoparticles, liquids, powders, or liposomes; troches that may be filled with liquids; chews; gels; rapidly dispersing dosage forms; films; ovules; sprays; and intrabuccal or mucosal adhesive patches. The liquid preparation includes suspensions, solutions, syrups, and elixirs. Such a preparation may be used as a filler for soft or hard capsules (e.g., made of gelatin or hydroxypropylmethylcellulose) and usually contains a carrier (e.g., water, ethanol, polyethylene glycol, propylene glycol, methylcellulose, or an appropriate oil) and one or more emulsifiers or suspending agents, or both. The liquid preparation may also be prepared by the reconstitution of a solid (e.g., from a sachet).

The pulmonary uptake can adopt an arbitrary form that permits uptake of caryophyllene. Examples thereof include respiration in space containing volatilized caryophyllene, and inhalation mediated by an inhalation tool. Examples of the inhalation mediated by an inhalation tool include inhalation mediated by a filter of an inhalation tool (e.g., the smoking of a tobacco provided with a caryophyllene-containing capsule in a filter).

The caryophyllene may be in a rapidly dissolving and rapidly disintegrating dosage form.

As for a tablet dosage form, the active pharmaceutical ingredient (API) constitutes approximately 1% by mass to approximately 80% by mass of the dosage form, more typically approximately 5% by mass to approximately 60% by mass of the dosage form, according to a dose. The tablet may contain, in addition to API, one or more disintegrants, binders, diluents, surfactants, glidants, lubricants, antioxidants, colorants, flavoring agents, preservatives, and/or corrigents. Examples of the disintegrant include starch sodium glycolate, sodium carboxymethylcellulose, calcium carboxymethylcellulose, croscarmellose sodium, crospovidone, polyvinylpyrrolidone, methylcellulose, microcrystalline cellulose, C1-6 alkyl-substituted hydroxypropylcellulose, starch, pregelatinized starch, and sodium alginate. In general, the disintegrant constitutes approximately 1% by mass to approximately 25% by mass or approximately 5% by mass to approximately 20% by mass of the dosage form.

The binder is generally used for imparting aggregability to a tablet preparation. Examples of the appropriate binder include microcrystalline cellulose, gelatin, sugars, polyethylene glycol, natural and synthetic rubbers, polyvinylpyrrolidone, pregelatinized starch, hydroxypropylcellulose, and hydroxypropylmethylcellulose. The tablet may also contain a diluent, for example, lactose (monohydrate, spray-dried monohydrate, or anhydrous), mannitol, xylitol, dextrose, sucrose, sorbitol, microcrystalline cellulose, starch, and/or calcium hydrogen phosphate dihydrate.

The tablet may also contain a surfactant, for example, sodium lauryl sulfate and/or polysorbate 80, and a glidant, for example, silicon dioxide and/or talc. The surfactant, if contained, may constitute approximately 0.2% by mass to approximately 5% by mass of the tablet, while the glidant may constitute approximately 0.2% by mass to approximately 1% by mas of the tablet.

The tablet may also contain a lubricant, for example, magnesium stearate, calcium stearate, zinc stearate, sodium stearyl fumarate, and/or a mixture of magnesium stearate and sodium lauryl sulfate. The lubricant may constitute approximately 0.25% by mass to approximately 10% by mass or approximately 0.5% by mass to approximately 3% by mass of the tablet.

The tablet may be formed by compressing a tablet blend either directly or by roller compression. Alternatively, the tablet blend or a portion of the blend may be subjected to a wet system, a dry system, or melt granulation, melt solidification, or extrusion before tableting. If necessary, one or more of the components may be classified by sieving or pulverization, or both before blending. The final dosage form may contain one or more layers and may be coated, uncoated, or a capsule.

The oral film that can be taken for use in humans or animals is in a soft water-soluble or water-swellable thin-film dosage form, which may rapidly dissolve or may be mucously adhesive. The film typically contains, in addition to API, one or more film-forming polymers, binders, wetting agents, plasticizers, stabilizers or emulsifiers, viscosity adjusters, and/or solvents. Examples of other film components may include antioxidants, colorants, flavoring agents and flavor enhancers, preservatives, salivary stimulants, cooling agents, cosolvents (including oils), emollients, extenders, antifoaming agents, surfactants, and corrigents. Some components of the formulation may exert two or more functions.

The amount of API in the film may depend on its solubility, in addition to administration requirements. Water-soluble API usually constitutes approximately 1% by mass to approximately 80% by mass of a non-solvent component (solute) in the film, or approximately 20% by mass to approximately 50% by mass of a solute in the film. Low soluble API may constitute a larger proportion of the composition, usually up to approximately 88% by mass of a non-solvent component in the film.

The film-forming polymer may be selected from natural polysaccharides, proteins, and synthetic hydrophilic colloids and usually constitutes approximately 0.01% by mass to approximately 99% by mass or approximately 30% by mass to approximately 80% by mass of the film.

The film dosage form is usually prepared by the evaporative drying of an aqueous thin film disposed on a removable support or support sheet. This operation may be performed in a drying furnace or a drying passage (e.g., a composite coating-drying apparatus), a freeze drying apparatus, or a vacuum furnace.

The preparation suitable for pulmonary administration includes solid, semisolid, and liquid systems, for example, tablets; and multiparticles or nanoparticles, liquids, powders, and liposomes. The pulmonary administration preferably employs a liquid preparation. The liquid preparation includes suspensions, solutions, syrups, and elixirs. Such a preparation can be used as a filler for soft capsules (including seamless capsules) or hard capsules. The pulmonary administration can be performed using a tobacco (tobacco filter), a smoking tool, an inhalation tool, or the like.

[Capsule]

The dosage form of the composition according to one aspect of the present invention may be a capsule. The capsule may be constituted by only a shell, and a constitution containing a core and a shell is preferred.

The capsule may be a soft capsule, a hard capsule, or the like, and may be a seamless capsule (capsule having no seam) or the like. Particularly, the capsule for tobaccos, etc. may be a seamless capsule.

In the capsule, the form of the composition is not particularly limited and may be, for example, a shell or a core, or both. Particularly, in a capsule (seamless capsule) having a core, the core (at least the core) may contain the composition.

The shell may usually contain a shell-forming component (film-forming agent or shell-forming agent). The shell-forming component is not particularly limited and can be appropriately selected according to the application of the capsule, etc. Examples thereof include polysaccharides (or their derivatives) {e.g., seaweed-derived polysaccharides [e.g., agar, carrageenan, alginic acid or its salts (e.g., metal salts such as alkali metal salts (sodium salt, potassium salt, etc.), alkaline earth metal salts (calcium salt, magnesium salt, etc.), iron salt, and tin salt), furcellaran, and curdlan], tree resin-derived polysaccharides (e.g., ghatti gum and gum arabic), microbe-derived polysaccharides (e.g., pullulan, welan gum, xanthan gum, and gellan gum), plant-derived polysaccharides (e.g., tragacanth gum, pectin, glucomannan, starch, polydextrose, dextrin, maltodextrin, cyclodextrin, and resistant dextrin), seed-derived polysaccharides [e.g., guar gum or its derivatives (e.g., hydroxypropyl guar gum, cationized guar gum, and guar gum degradation products (enzymatic degradation products of guar gum, etc.)), tara gum, tamarind seed gum, locust bean gum, psyllium seed gum, and flaxseed gum], fermented polysaccharides (e.g., diutan gum), cellulose derivatives (e.g., hydroxypropylcellulose, hydroxypropylmethylcellulose, methylcellulose, and carboxymethylcellulose), and chitosan}, synthetic resins (polyvinyl alcohol, etc.), proteins (e.g., gelatin, casein, and zein), and sugar alcohols (e.g., sorbitol, maltitol, lactitol, Palatinit, xylitol, mannitol, galactitol, and erythritol).

These shell-forming components may be used singly or in combinations of two or more thereof.

The shell-forming component may be capable of forming a hydrophilic colloid and may function as a plasticizer, a sweetener, dietary fibra, an extender, or the like, depending on its type. The shell-forming component to be used may be a commercially available product.

The shell may contain a plasticizer, a colorant, a sweetener, a fragrance, an antioxidant, an antiseptic, and the like.

The shell may contain, for example, a plasticizer for the adjustment of shell strength, etc. Examples of the plasticizer include polyhydric alcohols (e.g., (poly)alkylene glycol such as ethylene glycol, propylene glycol, polyethylene glycol, and polypropylene glycol; and polyol having three or more hydroxyl groups, such as glycerin), saccharides [e.g.,

monosaccharides (e.g., grape sugar, fruit sugar, glucose, and galactose), disaccharides (e.g., sucrose, maltose, trehalose, and coupling sugar), and oligosaccharides (e.g., maltooligosaccharide)], sugar alcohols (e.g., the sugar alcohols listed above, such as sorbitol, maltitol, lactitol, Palatinit, xylitol, mannitol, galactitol, and erythritol), polysaccharides or their derivatives [e.g., starch, starch derivatives (e.g., polydextrose, dextrin, maltodextrin, resistant dextrin, and cyclodextrin ($\alpha$, $\beta$, or $\gamma$)), and cellulose derivatives (e.g., hydroxypropylcellulose, hydroxypropylmethylcellulose, methylcellulose, and carboxymethylcellulose)], polyvinyl alcohol, and triacetin. These plasticizers may be used singly or in combinations of two or more thereof.

Sugar alcohols, starch, starch derivatives, and the like can also be used as shell-forming components as described above.

In the capsule having a core, the core may be solid or liquid, for example. Particularly, in, for example, the capsule having the composition of the present invention as contents, the core may be liquid. The liquid state also includes colloid, emulsion, and jelly states.

The core preferably contains caryophyllene and may further contain other components described above (disintegrant, binder, diluent, surfactant, glidant, lubricant, antioxidant, colorant, flavoring agent, preservative, corrigent, etc.).

The core may usually not dissolve (erode) the shell (or a portion in contact with the shell).

The size (diameter or average diameter) of the capsule (or shell) can be appropriately selected according to the type of the capsule, application, etc. The size may be, for example, 0.1 mm or larger, 0.5 mm or larger, 1 mm or larger, 1.5 mm or larger, or 2 mm or larger, and may be, for example, 30 mm or smaller, 25 mm or smaller, 20 mm or smaller, 18 mm or smaller, 15 mm or smaller, 12 mm or smaller, 10 mm or smaller, or 8 mm or smaller. Specific examples of the size of the capsule include, but are not limited to, 2.8 mm, 3.0 mm, 3.4 mm, 3.5 mm, and 4.0 mm.

In the capsule having a core, the shell ratio (ratio of the shell to the whole capsule (total amount of the shell and inclusions)) may be selected from, for example, the range of approximately 0.1 to 99% by mass (e.g., 0.5 to 95% by mass) and may be on the order of 1 to 90% by mass, preferably 1.5 to 80% by mass (e.g., 2 to 70% by mass), more preferably 2.5 to 60% by mass (e.g., 3 to 50% by mass).

In the capsule having a core, the thickness of the shell is not particularly limited and may be, for example, 1 to 200 $\mu$m, 3 to 150 $\mu$m, or 5 to 100 $\mu$m.

The capsule (e.g., a capsule having a core) may be breakable or disintegratable (e.g., easy-to-disintegrate or easy-to-break). In such a capsule, the breaking strength may be, for example, 100 g or more, 200 g or more, 300 g or more, 400 g or more, 500 g or more, 600 g or more, 700 g or more, 800 g or more, 900 g or more, 1000 g or more, though depending on the size of the capsule, etc.

The upper limit value of the breaking strength of the capsule is not particularly limited and may be, for example, 20000 g or less, 15000 g or less, 12000 g or less, or 10000 g or less.

For example, a value obtained by measurement at 22°C at 60% RH using a rheometer CR-500DX (measurement equipment, manufactured by Sun Scientific Co., Ltd.) followed by analysis with a Rheo data analyzer (Rheo Data Analyzer for Win, physical property data automatic analysis software, manufactured by Sun Scientific Co., Ltd.) can be used as the breaking strength (g).

In the capsule (e.g., a capsule having contents), the ratio of the breaking strength (g) to the outside diameter (mm) (breaking strength/outside diameter) is not particularly limited. The ratio may be, for example, 200 or more (e.g., more than 200), preferably 210 or more (e.g., 220 or more), more preferably 230 or more (e.g., 240 or more), and may be, for example, 250 or more, 300 or more, or 400 or more.

The upper limit of the ratio of the breaking strength to the outside diameter (breaking strength/outside diameter) is not particularly limited and may be, for example, 20000, 15000, 10000, 8000, 6000, or 5000.

Since the case where a capsule having large breaking strength is easy to break (e.g., the case of a large outside diameter), for example, is also assumed, such a ratio of the breaking strength to the outside diameter serves as an index that reflects the substantial ease of break of the capsule.

The breaking distance of the capsule may be, for example, 0.1 mm or larger, 0.2 mm or larger, 0.5 mm or larger, or 1.0 mm or larger, though depending on the outside diameter, etc.

The upper limit value of the breaking distance of the soft capsule is not particularly limited and may be, for example, 15 mm or smaller, 10 mm or smaller, or 8 mm or smaller.

The breaking distance can be measured with, for example, a rheometer CR-3000EX (manufactured by Sun Scientific Co., Ltd.).

In the capsule, the ratio of the breaking distance (mm) to the outside diameter (mm) (breaking distance/outside diameter) is not particularly limited. The ratio may be, for example, 0.1 or more, preferably 0.12 or more, more preferably 0.15 or more, and may be, for example, 0.18 or more or 0.2 or more.

The upper limit of the ratio of the breaking distance to the outside diameter (breaking distance/outside diameter) is not particularly limited and may be, for example, 1.0, 0.98, 0.97, 0.96, or 0.95.

The capsule may be used as it is, may be used in combination with an additional capsule, or may be used, for example, in a form incorporated in a filter as mentioned later, according to application, etc.

The additional capsule may be a capsule containing no caryophyllene. Examples thereof include capsules constituted by a core and a shell, neither of which contains caryophyllene.

A known method can be used as a method for producing the capsule (e.g., a seamless capsule). Examples of the production method include methods described in Japanese Patent No. 5047285, Japanese Translation of PCT International Application Publication No. 1998-506841, and Japanese Patent No. 5581446. Examples thereof include a dropping method into a liquid by a dropping system using double or more nozzles. By use of this method, a capsule content liquid is filled into a capsule shell, and then, the shell can be hardened and dried to produce a seamless capsule.

Examples of the application of such a capsule include medicaments, foods or drinks [e.g., foods with health claims (e.g., foods for specified health use and foods with nutrient function claims), and supplements], tobaccos, tobacco filters, smoking tools, and inhalation tools.

[Tobacco]

The composition according to one aspect of the present invention can be used in a tobacco. The composition can be contained in (attached to), for example, various portions of a tobacco (tobacco leaf, filter, etc.). Use in the tobacco permits pulmonary administration of caryophyllene.

Examples of such a form include forms in which the composition is contained in a tobacco leaf portion (e.g., tobaccos, cigars, pipes, smoke tubes, and smokeless tobaccos (e.g., chewing tobaccos and snuffing tobaccos (snus, snuff, etc.))), forms in which a filter comprising the composition is used in a tobacco, and forms in which a capsule (preferably a breakable (disintegratable) capsule) comprising the composition is placed in a filter. In the case of disposing a capsule in a filter, it is preferred that vaporized caryophyllene should be aspirated together with the smoke or the like of the tobacco by disintegrating the capsule at the time of smoking. The capsule for use in the tobacco is preferably constituted by a core (contents, a content liquid, or inclusions) and a shell (capsule shell).

The tobacco comprising the composition according to one aspect of the present invention is not particularly limited and may be a combustible tobacco (e.g., a tobacco, a cigar, a pipe, a smoke tube, and a bong) or a non-combustible tobacco (e.g., a heated tobacco (direct heating type, air heating type, etc.), and a smokeless tobacco).

More specific examples of the form include forms in which the composition (caryophyllene, menthol, etc.) that may be encapsulated is contained in a material to be inhaled (e.g., a liquid portion in a smoking tool) in a tobacco. Such use in the material to be inhaled permits efficient uptake of caryophyllene (and further, menthol) by pulmonary uptake.

For use in such a material to be inhaled (liquid, etc.), the composition of the present invention may comprise other components in addition to the caryophyllene (and further, an oil/fat and/or menthol) and may usually comprise a carrier [liquid carrier, for example, a polyhydric alcohol (e.g., glycerin and propylene glycol)] and the like. If necessary, the composition may further comprise a fragrance (in the form of a flavor liquid).

In the material to be inhaled (liquid, etc.), the ratio of the caryophyllene, etc. may be selected from the same range as above.

[Inhalation tool]

In the inhalation tool according to one aspect of the present invention, examples of the form of usage of the composition include, but are not particularly limited to, forms in which the composition is contained in (attached to) various portions of the inhalation tool. The inhalation tool may contain the composition in the form of a capsule containing the composition.

Use of the inhalation tool permits pulmonary administration of caryophyllene.

Examples of the inhalation tool include, but are not particularly limited to, smoking tools.

Examples of the smoking tool include heated tobaccos (vapor heating type, etc.), electronic tobaccos, pipes, smoke tubes, bongs (water pipes and water tobaccos), and vaporizers. The heated tobacco permits uptake of nicotine, and the electronic tobacco is free from nicotine. Examples of the heated tobacco include, but are not particularly limited to, IQOS (Philip Morris International Inc.), glo (British American Tobacco plc), Ploom S and Ploom TECH (Japan Tobacco Inc.), and PULZE (Imperial Brands pic). Examples of the electronic tobacco include, but are not particularly limited to, ego AIO (Joyetech Group) and ICE VAPE (Commonwealth).

More specific examples of the form include forms in which the composition (caryophyllene, menthol, etc.) that may be encapsulated is contained in a material to be inhaled (e.g., a liquid portion in a smoking tool) in an inhalation tool (e.g., a smoking tool such as heated tobaccos (vapor heating type, etc.), electronic tobaccos, and bongs). Such use in the material to be inhaled permits efficient uptake of caryophyllene (and further, menthol) by pulmonary uptake.

For use in such a material to be inhaled (liquid, etc.), the composition of the present invention may comprise other components in addition to the caryophyllene (and further, an oil/fat and/or menthol) and may usually comprise a carrier [liquid carrier, for example, a polyhydric alcohol (e.g., glycerin and propylene glycol)] and the like, as in the tobacco. If necessary, the composition may further comprise a fragrance (in the form of a flavor liquid).

In the material to be inhaled (liquid, etc.), the ratio of the caryophyllene, etc. may be selected from the same range as above.

[Filter]

In the filter, examples of the form of usage of the composition of the present invention include, but are not particularly limited to, forms in which the composition is contained in (attached to) various portions of a filter (filter material and filter member).

Particularly, such a filter may be a filter comprising a capsule (a filter incorporating a capsule or a filter constituted by a filter member incorporating a capsule).

Specifically, such a filter comprises, as a capsule, a capsule (first capsule) comprising the composition (caryophyllene, menthol, and an oil/fat). For example, any capsule described in the section about the capsule can be used as the first capsule. Particularly, the capsule (first capsule) is preferably a capsule constituted by a core and a shell, the core (contents) containing the composition of the present invention (or being the composition of the present invention).

Such a filter can comprise at least the first capsule as a capsule and may comprise a second capsule different from the first capsule.

The second capsule can be a capsule different from the first capsule. The second capsule may be, for example, a capsule having contents different from those of the first capsule.

Examples of such a second capsule include capsules constituted by a core and a shell, the core (and the shell) comprising at least any of a carrier and a fragrance (particularly, containing no caryophyllene).

Any capsule described in the section about the capsule can be used as the capsule contained in the filter as described above. Any capsule described in the section about the capsule can be used as the capsule containing no caryophyllene (second capsule, etc.) except for the presence or absence of caryophyllene.

The filter is not particularly limited and may be, for example, a filter for tobaccos, inhalation tools, air conditioners, or air purification systems.

Particularly, the filter comprising a capsule is suitable as a tobacco filter, etc. Such use in the tobacco filter, etc. permits efficient uptake of caryophyllene (and further, menthol) by pulmonary uptake.

The number of the capsule in the filter, etc. can be appropriately selected according to its application and may be one or may be two or more. For example, as described above, the filter may be constituted by only the first capsule (one type of capsule) and may further comprise the second capsule different from the first capsule. The number of the first capsule and the number of the second capsule may be one each or may be two or more each.

[Arterial stiffness]

In the present specification, the "arterial stiffness" refers to a loss of extensibility due to a hardened arterial vascular wall. Increased arterial stiffness prevents the pulse of heart rate from being absorbed by use of the extension of a vascular wall, and elevates a pulse wave velocity (PWV). PWV is measured using a carotid-femoral pulse wave velocity (cfPWV) or a brachial-ankle pulse wave velocity (baPWV), and a stiffness parameter $\beta$, an index for vascular wall elasticity, can be determined from these values.

Caryophyllene has a property of improving arterial stiffness or suppressing increase in arterial stiffness and can thereby be used in the treatment of vascular disease, the prevention of vascular disease, the treatment of hypertension, the prevention of hypertension, and the like.

[Vascular extracellular matrix]

Structures constituting the arterial vessel are divided into a cell layer composed of endothelial cells, smooth muscle cells, and the like, and an extracellular tissue composed of elastic fibras, collagenous fibras, and a membrane-like loose connective tissue. The vascular extracellular matrix is an extracellular tissue composed of extracellular fibras (elastic fibras, elastic lamina, and collagenous fibras), a loose connective tissue, and the like.

The degradation of vascular extracellular matrix facilitates the progression of vascular disease or hypertension. Caryophyllene has a property of suppressing the degradation of extracellular matrix and can thereby be used in the treatment of vascular disease, the prevention of vascular disease, the treatment of hypertension, the prevention of hypertension, and the like.

[Vascular fibra]

The arterial vessel has a three-layer structure of tunica intima composed of endothelial cells, tunica media composed of smooth muscle cells, elastic fibras, and collagenous fibras, and tunica adventitia composed of a loose connective tissue. The elastic fibras and the collagenous fibras are contained in vascular fibras. The vascular fibras are formed from collagen and elastin molecules, etc.

The destruction of vascular fibras facilitates the progression of vascular disease or hypertension. Caryophyllene has a property of suppressing the destruction of vascular fibras and can thereby be used in the treatment of vascular disease, the prevention of vascular disease, the treatment of hypertension, the prevention of hypertension, and the like.

[Vascular elastic lamina]

The vascular elastic lamina is an elastic fibrous tissue present between the tunica intima and the tunica media and between the tunica media and the tunica adventitia of the arterial vessel. The elastic lamina has a function of absorbing the pulse pressure of the blood vessel and is formed by the aggregation of elastic fibras which are extracellular fibras.

The destruction of vascular elastic lamina facilitates the progression of vascular disease or hypertension. Caryophyllene has a property of suppressing the destruction of vascular elastic lamina and can thereby be used in the treatment of vascular disease, the prevention of vascular disease, the treatment of hypertension, the prevention of hypertension, and the like.

[Matrix metalloprotease]

The matrix metalloprotease (MMP) is a proteolytic enzyme that typically degrades extracellular matrix formed from collagen, elastin, and the like, and has a metal ion coordinated at the active center. Secretory MMP-2 (gelatinase-A) and MMP-9 (gelatinase-B) play an important role in the degradation of vascular extracellular matrix. Among them, MMP-9 is an inducible enzyme and is activated by inflammatory stimulation.

The presence of MMP in excess causes the destruction of vascular elastic lamina or extracellular matrix and facilitates the progression of vascular disease or hypertension. Caryophyllene has a property of inhibiting MMP and can thereby be used in the treatment of vascular disease, the prevention of vascular disease, the treatment of hypertension, the prevention of hypertension, and the like.

The presence of MMP in excess causes the degradation of extracellular matrix in the skin, leading to a lack of vitality to the skin. Caryophyllene has a property of inhibiting MMP and can thereby maintain or improve the vitality to the skin and improve skin quality.

The presence of MMP in excess causes the degradation of extracellular matrix present in knee cartilage, leading to the easy onset of knee joint pain. Caryophyllene has a property of inhibiting MMP and can thereby suppress or improve knee joint pain.

[Vascular disease]

In the present specification, the "vascular disease" refers to a disease, disorder or injury of the blood vessel, mainly, artery and vein, which transports blood from and into the heart, the brain, and peripheral organs such as arms, legs, the kidney and the liver without limitations. Particularly, the "vascular disease" can refer to a disease of the coronary arterial and venous systems, the carotid and jugular systems, the aortic and caval systems, and peripheral arterial and venous systems. The disease that can be treated is any disease applicable to treatment with a therapeutic agent either as a single treatment protocol or as an aid to other manipulations such as surgical intervention. The disease can be a vascular lesion, atherosclerosis, vulnerable plaque, restenosis or peripheral arterial disease, without limitations. Examples of the vascular disease include aortic aneurysm, aortic dissection, triglyceride deposit cardiomyovasculopathy, and arterial and venous diseases of the kidney, the ileum, femoral region, popliteal space, the tibia and other vascular channel regions.

The peripheral vascular disease is generally caused by the structural change of the blood vessel caused by inflammation and injury to tissues. A subset of peripheral vascular disease is peripheral arterial disease (PAD). PAD is a condition similar to carotid arterial disease and coronary arterial disease in terms of being caused by fat accumulation constructed in the lining or tunica intima of an arterial wall. The occlusion of carotid artery restricts blood flow to the brain, and the occlusion of coronary artery restricts blood flow to the heart. Likewise, the occlusion of peripheral artery might restrict blood flow to the kidney, the stomach, arms, legs and feet. Particularly, at present, the peripheral vascular disease often refers to vascular disease of superficial femoral artery.

[Hypertension]

In the present specification, the "hypertension" means a high level of systolic blood pressure and/or diastolic blood pressure. For example, a subject having hypertension may have a systolic blood pressure of 120 mmHg or higher (e.g., 140 mmHg higher or ≥160 mmHg or higher) and/or a diastolic blood pressure of 80 mmHg or higher (e.g., 90 mmHg or higher or ≥100 mmHg or higher).

[Examples]

Hereinafter, the present invention will be described further specifically with reference to Examples, etc. However, the present invention is not limited by these Examples, etc. Examples of the present specification disclose a prophylactic effect and/or a therapeutic effect on adverse effects on blood vessels or the like induced by nicotine uptake. However, these effects are not limited by the adverse effects ascribable to nicotine uptake. For example, a vascular disease therapeutic effect, a vascular disease prophylactic effect, a hypertension therapeutic effect, and a hypertension prophylactic effect on diseases such as vascular disease or hypertension induced by other factors such as age, sex, hypertension, arteriosclerosis, and oxidative stress can also be expected.

Examples of the present specification list, as the pulmonary uptake of β-caryophyllene, respiration in space containing volatilized β-caryophyllene, the smoking of a tobacco provided with a β-caryophyllene-containing capsule in a filter, and the like. However, the pulmonary uptake of β-caryophyllene is not limited thereto, and any form that eventually permits uptake of β-caryophyllene can be adopted. Examples thereof also include pulmonary uptake with an inhalation tool for the inhalation of β-caryophyllene volatilized without firing, and the pulmonary uptake of β-caryophyllene scattered into space with an aromatic substance containing the β-caryophyllene.

The oral uptake also includes the oral uptake of β-caryophyllene mixed into a food or a drink.

[Example 1] Pulmonary uptake of β-caryophyllene (bioavailability)

In this Example, an experimental system of pulmonary β-caryophyllene uptake models using mice was used. Specifically, 4-week-old Slc:ddY mice were obtained from SHIMIZU Laboratory Supplies Co., Ltd. and given commercially available diets and water by discretionary uptake. The mice were raised at 25°C ± 1°C in a 12-hour shift of the light-dark cycle according to a standard approach and acclimatized for 5 days to prepare laboratory mice.

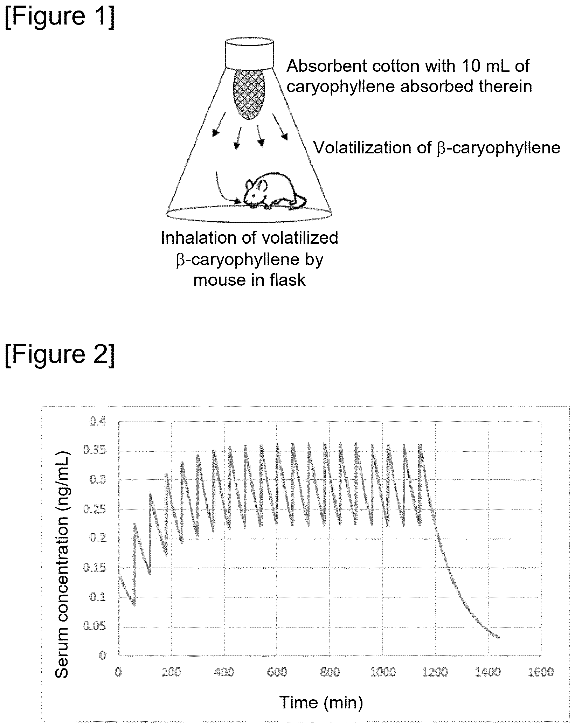

In order to allow the mice to inhale β-caryophyllene for pulmonary uptake, absorbent cotton infiltrated with 10 mL of β-caryophyllene was hung in the upper part of a 5 L flask and left for a given time to provide a state with a spatial β-caryophyllene concentration of 3.75 μg/100 mL inside the flask (see Figure 1) (hereinafter, referred to as a "caryophyllene-filled flask"). Since the respiratory volume of the mice is 24 mL/min (Jikken Dobutsu Gaku Kakuron (Details of Experimental Zoology in English), Yoshio Tajima ed., 1972, Asakura Publishing Co., Ltd.), 1440 mL of air was inhaled for 60 minutes. Thus, the pulmonary intake of β-caryophyllene by each mouse placed in the flask was 54 μg in 1 hour.

The mice were placed in the caryophyllene-filled flasks so that the mice inhaled β-caryophyllene. Specifically, the following three mice were provided.

(1) Mouse that was not placed in the caryophyllene-filled flask (no exposure)
(2) Mouse placed in the caryophyllene-filled flask for 1 minute and thereby exposed
(3) Mouse placed in the caryophyllene-filled flask for 60 minutes and thereby exposed

These mice were anesthetized and dissected 10 minutes later to obtain serum (approximately 1 mL), the liver (whole left lobe), and the whole brain (cerebrum and cerebellum) of each mouse. The serum and these organs were each ground in a mortar, and β-caryophyllene was extracted with acetone. The extracts were volatilized, and after adsorption through a Tenax tube (Gerstel K.K., TDU Tube Tenax TA), a β-caryophyllene concentration was quantified by use of GC/MS analysis (gas chromatography mass spectrometry) (Agilent Technologies Inc., 7890B/5977B GC/MSD). The concentrations of β-caryophyllene contained in serum, the liver and the whole brain for the three mice were as described in Table 1.

[Table 1]

Table 1

| | Serum (ng/mL) | Liver (ng/g) | Whole brain (ng/g) |
|---|---|---|---|
| (1) No exposure | 0 | 0 | 0 |
| (2) 1-min exposure | 1.6 | 28 | 44 |
| (3) 60-min exposure | 102 | 1127 | 1325 |

It was found from the results of Table 1 that β-caryophyllene contained in air was brought into the body by pulmonary uptake through respiration and transferred to at least serum, the liver and the brain.

[Example 2] Pulmonary uptake of β-caryophyllene (bioavailability, metabolism and excretion)

In the same manner as in Example 1, laboratory mice were provided, and the laboratory mice were placed in the caryophyllene-filled flasks so that the mice inhaled β-caryophyllene. Specifically, the following five mice were provided.

(1) Mouse that was not placed in the caryophyllene-filled flask (no exposure)
(2) Mouse placed in the caryophyllene-filled flask for 60 minutes and thereby exposed
(3) Mouse placed in the caryophyllene-filled flask for 60 minutes and thereby exposed, then taken out of the flask, and left in cleaned air for 60 minutes
(4) Mouse placed in the caryophyllene-filled flask for 60 minutes and thereby exposed, then taken out of the flask, and left in cleaned air for 180 minutes
(5) Mouse placed in the caryophyllene-filled flask for 60 minutes and thereby exposed, then taken out of the flask, and left in cleaned air for 24 hours

In the same manner as in Example 1, these five mice were anesthetized and dissected 10 minutes later to obtain serum (approximately 1 mL), the liver (whole left lobe), and the whole brain (cerebrum and cerebellum) of each mouse. The concentrations of β-caryophyllene contained in serum, the liver and the whole brain for the five mice were as described in Table 2.

[Table 2]

Table 2

| | Serum (ng/mL) | Liver (ng/g) | Whole brain (ng/g) |
|---|---|---|---|
| (1) No exposure | 0 | 0 | 0 |
| (2) 60-min exposure | 142 | 298 | 283 |
| (3) 60-min exposure + 60-min leaving | 77 | 170 | 307 |
| (4) 60-min exposure + 180-min leaving | 42 | 69 | 172 |
| (5) 60-min exposure + 24-h leaving | 0 | 33 | 37 |

From the results of Table 2, when the mice were allowed to inhale β-caryophyllene for 60 minutes and then left in cleaned air, the β-caryophyllene concentration was decreased in all of serum, the liver and the whole brain. Particularly, in serum and an organ having highly water solubility, such as the liver, the β-caryophyllene concentration was markedly decreased as the time passed. In the brain, the concentration of β-caryophyllene was slightly increased for the mouse left in cleaned air for 60 minutes after the completion of exposure to β-caryophyllene for 60 minutes, whereas the concentration of β-caryophyllene was markedly decreased for the mice left for 180 minutes or for 24 hours. It was thus found that β-caryophyllene is metabolized and excreted, without being excessively accumulated in the body, after uptake into the body and is thus a highly safe component.

The β-caryophyllene concentration in the body is considered to be exponentially decreased and can be approximated according to the following expressions.

[Expression 1]

- Serum concentration: $y = 142 \times \exp\frac{t}{123}$ Half-life of 85.4 min

- Liver concentration: $y = 298 \times \exp\frac{t}{115}$ Half-life of 79.8 min

- Brain concentration: $y = 301 \times \exp\frac{t}{457}$ Half-life of 316.5 min

[Example 3] Capsule-containing tobacco filter

(1) Weight of β-caryophyllene per tobacco contained in mainstream smoke

In order to produce capsule-containing tobacco filters, two types of easy-to-disintegrate capsules given below were prepared as the capsules to be disposed in the filters by the dropping method. The weights of capsule content liquids were both set to 19.3 mg.

Capsule 1: 100% by mass of β-caryophyllene
Capsule 2: 15% by mass of β-caryophyllene, 15% by mass of L-menthol, and 70% by mass of MCT

The two types of capsules prepared were each inserted to the central part of the tobacco filter of a tobacco (CORESTA CM9 manufactured by Borgwaldt GmbH). Then, the capsule in the filter was disintegrated by pressurization, and the tobacco was lit and smoked. The weight of β-caryophyllene per tobacco contained in mainstream smoke upon inhalation was measured. As for a tobacco provided aside from these, the weight of β-caryophyllene per tobacco contained in mainstream smoke was measured without disintegrating the capsule. Specifically, vapor components and particulate components of three tobaccos were adsorbed to the glass filter of a smoking machine, and the amount of β-caryophyllene volatilized per tobacco was determined using GC/MS with reference to ISO 10315. The results are as described in Table 3. The smoking machine used was Linear Smoking Machine (LM2) manufactured by Borgwaldt GmbH, and the smoking was performed in accordance with ISO 3308.

[Table 3]

Table 3

| | Amount of β-caryophyllene (mg) |
| --- | --- |
| Tobacco in which capsule was contained in filter but was not disintegrated | Below detection limit |
| Tobacco in which capsule 1 was contained in filter and disintegrated before smoking | 2.99+0.009 |
| Tobacco in which capsule 2 was contained in filter and disintegrated before smoking | 0.29+0.03 |

As shown in Table 3, when mainstream smoke was inhaled by lighting the tobacco having the tobacco filter containing the capsule 1, approximately 3 mg of volatilized β-caryophyllene was able to be inhaled. When mainstream smoke was inhaled by lighting the tobacco having the tobacco filter containing the capsule 2, approximately 0.3 mg of volatilized β-caryophyllene was able to be inhaled.

(2) Weight of β-caryophyllene remaining in filter

In order to produce capsule-containing tobacco filters, three types of easy-to-disintegrate capsules given below were prepared as the capsules to be disposed in the filters by the dropping method. The weights of capsule content liquids were all set to 19.3 mg.

Capsule 3: 30% by mass of β-caryophyllene and 70% by mass of MCT
Capsule 4: 15% by mass of β-caryophyllene and 85% by mass of MCT
Capsule 5: 5% by mass of β-caryophyllene and 95% by mass of MCT

The three types of capsules prepared were each inserted to the central part of the tobacco filter of a commercially available tobacco.

Tobacco using capsule 3

Tobacco 1: Natural American Spirit Organic Mint ONE (nicotine: 0.1 mg, tar: 1 mg)
Tobacco 2: Hi-Lite (nicotine: 1.4 mg, tar: 17 mg) Tobacco using capsule 4
Tobacco 3: Lark Extra 3 mg 100 Box (nicotine: 0.2 mg, tar: 3 mg)
Tobacco 4: Marlboro Medium Box (nicotine: 0.7 mg, tar: 8 mg)

Tobacco using capsule 5

Tobacco 5: MEVIUS Light Box (nicotine: 0.7 mg, tar: 8 mg)
Tobacco 6: Kool Light Box (nicotine: 0.4 mg, tar: 5 mg)

Then, the capsule in the filter was disintegrated by pressurization, and the tobacco was lit and smoked in 8 puffs by a human. The weight of $\beta$-caryophyllene remaining in the filter was measured. Specifically, the weight of $\beta$-caryophyllene remaining per filter was determined using GC/MS with reference to ISO 10315. The results are as described in Table 4.

[Table 4]

Table 4

|  | Tobacco 1 | Tobacco 2 | Tobacco 3 | Tobacco 4 | Tobacco 5 | Tobacco 6 |
|---|---|---|---|---|---|---|
| Amount of $\beta$-caryophyllene added in capsule (mg) | 5.79 | | 2.90 | | 0.91 | |
| Weight of $\beta$-caryophyllene remaining in filter (mg) | 0.0284 | 0.0279 | 0.0117 | 0.0104 | 0.0038 | 0.0058 |

From the results of Table 4, the amount of $\beta$-caryophyllene remaining in the filter was only less than 1% of the amount of the $\beta$-caryophyllene added to the capsule. It was thus found that 99% or more of $\beta$-caryophyllene was volatilized and inhaled by smoking.

(3) Weight of $\beta$-caryophyllene contained in exhaled smoke

The prepared capsule 2 was inserted to the central part of the tobacco filter of a tobacco (CORESTA CM9 manufactured by Borgwaldt GmbH) (tobacco 7). Then, the capsule in the filter was disintegrated by pressurization, and the tobacco was lit and smoked. Breath exhaled after smoking was recovered into a bag, and volatile organic compounds contained in exhaled smoke were adsorbed to a Tenax tube using a measuring pump (150 mL/min). Then, the amount of $\beta$-caryophyllene contained in the exhaled smoke upon smoking of one tobacco was determined by use of GC/MS. The results are as described in Table 5.

[Table 5]

Table 5

|  | Tobacco 7 |
|---|---|
| Amount of $\beta$-caryophyllene added in capsule (mg) | 2.90 |
| Weight of $\beta$-caryophyllene contained in exhaled smoke per tobacco ($\mu$g) | 1.9$\pm$0.3 |

As described above, the weight of $\beta$-caryophyllene exhaled was only less than 0.1% of the amount of the $\beta$-caryophyllene added to the capsule. This suggests that 99.9% or more of $\beta$-caryophyllene inhaled by smoking was brought into the body.

[Example 4] Oral uptake of β-caryophyllene (bioavailability)

In the same manner as in Example 1, laboratory mice were provided, and an experiment of oral β-caryophyllene uptake models was conducted. β-caryophyllene was orally administered at 20 μg per g of body weight to the laboratory mice using a sonde. Since the body weights of the laboratory mice were 25 g, 500 μg of β-caryophyllene was administered to each mouse. In Example 1, the pulmonary intake of β-caryophyllene by each laboratory mouse was 54 μg in 1 hour. Therefore, β-caryophyllene was able to be taken by oral administration in an amount of approximately 10 times the pulmonary intake.

In the same manner as in Example 1, caryophyllene-filled flasks were provided, and a mouse placed in the flask for 60 minutes and thereby exposed, and a mouse that underwent neither oral administration nor pulmonary uptake of β-caryophyllene were provided. In this manner, the following mice were provided.

(1) Mouse neither placed in the caryophyllene-filled flask nor orally given β-caryophyllene
(2) Mouse placed in the caryophyllene-filled flask for 60 minutes and thereby exposed
(3) Mouse orally given β-caryophyllene

The mouse orally given β-caryophyllene was anesthetized 30 minutes after oral administration, and then, the mouse was dissected to obtain serum, thoracic aorta and abdominal aorta. The mouse placed in the caryophyllene-filled flask for 60 minutes and thereby exposed was dissected 10 minutes after anesthesia in the same manner as in Example 1 to obtain serum, thoracic aorta and abdominal aorta.

The concentrations of β-caryophyllene contained in serum, thoracic aorta and abdominal aorta for these three mice are as described in Table 6.

[Table 6]

Table 6

|  | Serum (ng/mL) | Thoracic aorta (ng/g) | Abdominal aorta (ng/g) |
|---|---|---|---|
| (1) No exposure | 0 | 0 | 0 |
| (2) 60-min exposure | 34 | 751 | 403 |
| (3) Oral administration | 8.8 | 1322 | 210 |

As shown in Table 6, difference in β-caryophyllene concentration in serum, thoracic aorta, or abdominal aorta was within 4 times between in the case of exposure for 60 minutes in the caryophyllene-filled flask (pulmonary uptake) and in the case of oral administration of β-caryophyllene. In consideration of the fact that β-caryophyllene was taken by oral administration in an amount of approximately 10 times the pulmonary intake, bioavailability was approximately 10 times higher in the pulmonary uptake compared with the oral administration.

[Examples 5 to 7] Suppression of destruction of elastic fibra and inhibition of matrix metalloprotease (MMP) in aortic tissue by β-caryophyllene

1 Harvest of rat abdominal aorta

7-week-old male Sprague-Dawley rats were obtained from Japan SLC, Inc. and given commercially available diets and water by discretionary uptake. The rats were raised at 25°C $\pm$ 1°C in a 12-hour shift of the light-dark cycle according to a standard approach to prepare laboratory rats.

After an acclimatization period for 4 days, the laboratory rats were each dissected to obtain abdominal aorta. Unnecessary surrounding tissues were removed from the harvested abdominal aorta, which was then dipped in a medium for aorta culture. The medium for aorta culture was prepared by adding 45 mL of 10% FBS, 1 mL of a penicillin-streptomycin solution, 10 mL of 200 mM L-glutamic acid, 10 mL of a glucose solution, and 22 mL of 7.5% NaHCOs to DMEM medium.

2 Preparation of sample

The harvested abdominal aorta was prepared into a control group supplemented with neither nicotine nor β-caryophyllene (Comparative Example 1), a nicotine group supplemented with only nicotine (Comparative Example 2), a β-caryophyllene 100 group supplemented with nicotine and β-caryophyllene such that the β-caryophyllene concentration was 100 nM (Example 5), a β-caryophyllene 200 group supplemented with nicotine and β-caryophyllene such that the β-caryophyllene concentration was 200 nM (Example 6), and a β-caryophyllene 500 group supplemented with nicotine and β-caryophyllene such that the β-caryophyllene concentration was 500 nM (Example 7). These abdominal aorta specimens were cultured in a medium for aorta culture under conditions of 37°C and 5% $CO_2$ for 24 hours.

Sampling was performed in the same manner as the approach described in Non Patent Literature 2 (Hirona Kugo, Nobuhiro Zaima, Megumi Onozato, Chie Miyamoto, Keisuke Hashimoto, Kenichi Yanagimoto, Tatsuya Moriyama, Food Funct., 2017, 8, 2829-2835). Specifically, the abdominal aorta thus cultured was recovered, dipped in 4% paraformaldehyde, fixed overnight at 4°C, and then embedded in paraffin.

3 Measurement of rate of destruction of elastic fibra and measurement of matrix metalloprotease (MMP)-positive area

The rate of destruction of elastic fibras of each sample was measured in the same manner as the approach reported in Non Patent Literature 3. Specifically, the aortic wall was stained by Elastica van Gieson (EVG) staining and immunohistochemical staining. The quantitative analysis of the histochemical stain was carried out using Imaged software (National Institutes of Health, Bethesda, Maryland, USA).

Since an elastic lamina waveform is EVG-stained, the destruction of the vascular wall was estimated from the ratio of its area. The rate of destruction of the wavy configuration of elastic lamina was calculated by dividing a destroyed region (indicated by flattening and fragmentation of elastic lamina) by the whole region of the elastic lamina. When the rate of destruction of the elastic lamina waveform was less than 30%, the sample was evaluated as having a state in which the vascular wall was not destroyed (evaluation A). When the rate of destruction of the elastic lamina waveform was 30% or more, the sample was evaluated as having a state in which the vascular wall was destroyed (evaluation B). The results about each sample are as described in Table 7.

[Table 7]

Table 7

|  | Rate of destruction (%) | Evaluation |
| --- | --- | --- |
| Comparative Example 1 | 15 | A |
| Comparative Example 2 | 43 | B |
| Example 5 | 17 | A |
| Example 6 | 20 | A |
| Example 7 | 22 | A |

As shown in Table 7, the rate of destruction of the wavy configuration of elastic lamina was highest in Comparative Example 2 in which only nicotine was added, whereas the addition of β-caryophyllene together with nicotine reduced the rate of destruction, which got close to that of Comparative Example 1 (control) in which no nicotine was added.

The immunohistochemical staining of each sample was performed in the same manner as the approach described in Non Patent Literature 2. Specifically, the tissue section of the sample was washed with phosphate-buffered saline containing 1% Triton-X 100, incubated with 10% oxalic acid for 1 hour, and then stained using an antibody, followed by the measurement of an MMP-positive area. Specifically, the ratio (%) of a stained area to the whole area photographed was measured. A positive area of 1.50 or less was given evaluation A, and a positive area of more than 1.50 was given evaluation B. The results about each sample are as described in Table 8.

[Table 8]

Table 8

|  | Positive area (%) | Evaluation |
|---|---|---|
| Comparative Example 1 | 0.79 | A |
| Comparative Example 2 | 2.90 | B |
| Example 5 | 0.35 | A |
| Example 6 | 0.96 | A |
| Example 7 | 0.36 | A |

As shown in Table 8, the MMP-positive area was highest in Comparative Example 2 in which only nicotine was added, whereas the addition of β-caryophyllene together with nicotine decreased the MMP-positive area and the positive areas of Examples 1 and 3 were lower than that of Comparative Example 1 in which no nicotine was added.

[Example 8] Recovery of elastic fibra in aortic tissue by β-caryophyllene

1 Harvest of rat abdominal aorta

In the same manner as in Examples 5 to 7, laboratory rats were provided, and abdominal aorta was harvested from each of these rats to make preparations for sample production.

2 Production of sample

The following four samples were produced.

(1) Control group of the harvested abdominal aorta cultured in a medium for aorta culture under conditions of 37°C and 5% $CO_2$ for 24 hours (Comparative Example 3)
(2) Nicotine group A of the harvested abdominal aorta cultured in a medium for aorta culture supplemented with nicotine under conditions of 37°C and 5% $CO_2$ for 24 hours

(Comparative Example 4)

(3) Nicotine group B of the harvested abdominal aorta cultured in a medium for aorta culture supplemented with nicotine under conditions of 37°C and 5% $CO_2$ for 24 hours and further cultured in a nicotine-free medium for 24 hours (Comparative Example 5)
(4) β-caryophyllene 100 group of the harvested abdominal aorta cultured in a medium for aorta culture supplemented with nicotine under conditions of 37°C and 5% $CO_2$ for 24 hours and further cultured under conditions of 37°C and 5% $CO_2$ for 24 hours in a medium supplemented with β-caryophyllene such that the β-caryophyllene concentration was 100 nM (Example 8)

Sampling was performed in the same manner as in Examples 5 to 7.

3 Measurement of rate of destruction of elastic fibra

The rate of destruction of elastic fibras was measured in the same manner as in Examples 5 to 7. A rate of destruction of 35% or less was given evaluation A, and a rate of destruction of more than 35% was given B. The results about each sample are as described in Table 9.

[Table 9]

Table 9

|  | Rate of destruction (%) | Evaluation |
|---|---|---|
| Comparative Example 3 | 16 | A |
| Comparative Example 4 | 54 | B |
| Comparative Example 5 | 58 | B |
| Example 8 | 18 | A |

As shown in Table 9, the rate of destruction of the wavy configuration of elastic lamina was increased from 16% to 54% by the addition of nicotine (Comparative Examples 3 and 4), whereas the subsequent addition of β-caryophyllene decreased the rate of destruction to the same level (18%) as in the control before addition of nicotine (Comparative Example 3).

From Example 4, when a mouse takes 54 μg of β-caryophyllene by inhalation, the β-caryophyllene concentration in the thoracic or abdominal aorta is 400 to 700 ng/g, i.e., 1.96 to 3.43 nmol/g (molecular weight of β-caryophyllene: 204.36).

From Table 7, β-caryophyllene was able to be confirmed to have a suppressive effect on the destruction of vascular elastic fibras at 100 nM = 0.1 nmol/g. It is therefore expected that an inhalation intake of 1/40 to 1/20 also produces a suppressive effect on the destruction of vascular elastic fibras in rats or mice.

[Example 9] Pulmonary uptake of β-caryophyllene (suppression of destruction of elastic fibra and inhibition of matrix metalloprotease in aortic tissue by β-caryophyllene)

1 Raising of mouse and harvest of abdominal aorta

Four-week-old male ddy mice (body weight: 18 g) were obtained from Japan SLC, Inc. and given commercially available diets and water by discretionary uptake. The mice were raised at 25°C ± 1°C in a 12-hour shift of the light-dark cycle according to a standard approach.

Then, the mice were divided into a nicotine + caryophyllene group (Example 9), a nicotine group (Comparative Example 8), a caryophyllene group (Comparative Example 7), and a control group (Comparative Example 6) each involving one mouse, and raised for 7 days. Specifically, for the nicotine + caryophyllene group (Example 9) and the caryophyllene group (Comparative Example 7), as shown in Figure 1, absorbent cotton infiltrated with 10 mL of β-caryophyllene was hung in a 5 L flask and left for 10 minutes, and the mouse was raised in the flask filled with β-caryophyllene for 1 hour each for 6 days among 14 days. The mouse was raised in the same cage as that for the control group (Comparative Example 7) except for the time when the mouse took β-caryophyllene by inhalation. For the nicotine + caryophyllene group (Example 5) and the nicotine group (Comparative Example 8), the mouse was given an aqueous nicotine solution having a concentration of 0.5 mg/mL by discretionary uptake. For the control group (Comparative Example 6), the mouse was given nicotine-free water by discretionary uptake in normal air.

After the test, thoracic aorta and abdominal aorta were obtained by dissection. Each harvested aorta was Elastica van Gieson (EVG)-stained to measure and evaluate the rate of destruction of elastic lamina.

2 Measurement of rate of destruction of elastic fibra

The rate of destruction of elastic fibras was measured in the same manner as in Examples 5 to 7. The results about each sample are as described in Table 10.

[Table 10]

Table 10

|  | Rate of destruction (%) | Evaluation |
|---|---|---|
| Comparative Example 6 | 10% | A |
| Comparative Example 7 | 11% | A |
| Comparative Example 8 | 42% | B |

(continued)

|  | Rate of destruction (%) | Evaluation |
|---|---|---|
| Example 9 | 15% | A |

In Comparative Example 8, nicotine destroyed 42% of the elastic lamina. By contrast, in Example 5 involving the uptake of nicotine and caryophyllene, only 15% thereof was destroyed, and the rate of destruction was equivalent to that of Comparative Examples 6 and 7 (10% and 11%) involving no nicotine uptake. Thus, the uptake of β-caryophyllene suppressed the destruction of vascular elastic lamina by nicotine.

As described above, the uptake of β-caryophyllene prevented the nicotine-induced destruction of elastic lamina, suggesting the absence of decrease in vascular wall thickness and decrease in the amount of collagenous fibras contained in elastic lamina due to the destruction of elastic lamina as well as increase in MMP-2 and MMP-9 (which destroy elastic lamina)-positive ratios.

[Example 10] Pulmonary uptake of β-caryophyllene (improvement in arterial stiffness, suppression of increase in arterial stiffness)

1 Preparation of capsule-containing tobaccos

In the same manner as for the capsules 1 to 5 in Example 3, a capsule 6 containing 100% by mass of MCT was produced. Then, the capsules 3 and 4 prepared in Example 3 and the capsule 6 were each inserted to the central part of the tobacco filter of a commercially available tobacco (the weights of capsule content liquids were each set to 19.3 mg) as follows.

Tobacco using capsule 3 (Examples 10-1 to 10-4)

Tobacco 7: Kool Light Box (nicotine: 0.4 mg, tar: 5 mg)
Tobacco 8: MEVIUS Light Box (nicotine: 0.7 mg, tar: 8 mg)
Tobacco 9: Winston CASTER WHITE 5 Box (nicotine: 0.4 mg, tar: 5 mg)
Tobacco 10: Cool Light Box (nicotine: 0.4 mg, tar: 5 mg)

Tobacco using capsule 4 (Examples 10-5 to 10-9)

Tobacco 11: Peace AROMA Infinity (nicotine: 0.7 mg, tar: 8 mg)
Tobacco 12: Lark Extra 3 mg 100 Box (nicotine: 0.2 mg, tar: 3 mg)
Tobacco 13: VIRGINIA S DUO Menthol 10's (nicotine: 0.1 mg, tar: 1 mg)
Tobacco 14: SevenStars 7 Box (nicotine: 0.6 mg, tar: 7 mg)
Tobacco 15: Marlboro Medium Box (nicotine: 0.7 mg, tar: 8 mg)

Tobacco using capsule 6 (Comparative Examples 9-1 to 9-5)

Tobacco 16: Lark Ultra 1 mg KS Box (nicotine: 0.1 mg, tar: 1 mg)
Tobacco 17: Winston CABIN RED 8 100's Box (nicotine: 0.7 mg, tar: 8 mg)
Tobacco 18: Lark Super Mild 100's Box (nicotine: 0.5 mg, tar: 6 mg)
Tobacco 19: SevenStars Box (nicotine: 1.2 mg, tar: 14 mg)
Tobacco 20: Natural American Spirit Organic Mint ONE (nicotine: 0.1 mg, tar: 1 mg)

Healthy subjects were allowed to smoke a predetermined number of tobaccos per day for 5 weeks, where the capsule in the filter was disintegrated by pressurization, and the tobacco was lit and smoked by each healthy subject, and left and right brachio-ankle pulse wave velocities (PWVs) (cm/s) were measured and the stiffness parameters β were determined for each subject, where stiffness parameters β were calculated from the following expression (Shirai, K.; Utino, J.; Otsuka, K.; Takata, M., J. Atheroscler Thromb., 2006, 13, 101-107.):

[Expression 2]

$$\beta = \left(\ln \frac{P_s}{P_d}\right) \times \frac{2\rho}{\Delta P} \times PW^2$$

wherein $P_s$ denotes systolic blood pressure, $P_d$ denotes diastolic blood pressure, PWV denotes pulse wave velocity between a brachium and an ankle, $\rho$ denotes blood density, and $\Delta P$ denotes pulse pressure.
PWV, systolic blood pressure, diastolic blood pressure, blood density, and pulse pressure were measured by using a BP-203RPE II manufactured by OMRON COLIN Co., Ltd.

Specifically, the PWVs and stiffness parameters $\beta$ of each subject was measured before initiation of 5-week smoking experiment (Initial PWV, Initial stiffness parameter $\beta$). Thereafter, each subject was allowed to continuously smoke tobaccos in Number of tobaccos smoked per day shown in Tables 11 to 13 for 5 weeks. Five weeks after the initiation of experiment, the PWVs and stiffness parameters $\beta$ were measured again (PWV after 5 weeks, Stiffness parameter $\beta$ after 5 weeks), and the change rates of these values were also calculated. The results obtained were as shown in Tables 11 to 13.

[Table 11]

Table 11 Tobaccos 7 to 10 (capsule 3)

| Example | 10-1 | 10-2 | 10-3 | 10-4 |
|---|---|---|---|---|
| Sex of subject | Male | Male | Male | Male |
| Age of subject | 49 | 35 | 33 | 35 |
| Tobacco smoked | Tobacco 7 | Tobacco 8 | Tobacco 9 | Tobacco 10 |
| Number of tobaccos smoked per day | 6 | 6 | 8 | 12 |
| Initial PWV (right) | 1664 | 1615 | 1600 | 1514 |
| PWV after 5 weeks (right) | 1601 | 1523 | 1622 | 1404 |
| Change rate of PWV (right) | 96.2% | 94.3% | 101.4% | 92.7% |
| Initial PWV (left) | 1540 | 1546 | 1579 | 1501 |
| PWV after 5 weeks (left) | 1490 | 1458 | 1486 | 1386 |
| Change rate of PWV (left) | 96.8% | 94.3% | 94.1% | 92.3% |
| Initial stiffness parameter $\beta$ (right) | 6.01 | 4.32 | 4.88 | 4.74 |
| Stiffness parameter $\beta$ after 5 weeks (right) | 4.96 | 3.70 | 5.16 | 3.98 |
| Change rate of stiffness parameter $\beta$ (right) | 82.5% | 85.7% | 105.6% | 84.0% |
| Initial stiffness parameter $\beta$ (left) | 4.93 | 3.87 | 4.74 | 4.36 |
| Stiffness parameter $\beta$ after 5 weeks (left) | 4.38 | 3.43 | 4.33 | 3.83 |
| Change rate of stiffness parameter $\beta$ (left) | 88.8% | 88.7% | 91.4% | 87.9% |

[Table 12]

Table 12 Tobaccos 11 to 15 (capsule 4)

| Example | 10-5 | 10-6 | 10-7 | 10-8 | 10-9 |
|---|---|---|---|---|---|
| Sex of subject | Male | Female | Male | Male | Male |
| Age of subject | 32 | 55 | 35 | 32 | 43 |
| Tobacco smoked | Tobacco 11 | Tobacco 12 | Tobacco 13 | Tobacco 14 | Tobacco 15 |
| Number of tobaccos smoked per day | 4 | 16 | 7 | 15 | 9 |
| Initial PWV (right) | 1512 | 1589 | 1447 | 1426 | 1598 |
| PWV after 5 weeks (right) | 1311 | 1491 | 1216 | 1382 | 1466 |
| Change rate of PWV (right) | 86.7% | 93.8% | 84.0% | 96.9% | 91.7% |
| Initial PWV (left) | 1569 | 1592 | 1391 | 1519 | 1666 |
| PWV after 5 weeks (left) | 1245 | 1593 | 1208 | 1316 | 1385 |
| Change rate of PWV (left) | 79.3% | 100.1% | 86.8% | 86.6% | 83.1% |
| Initial stiffness parameter $\beta$ (right) | 5.03 | 4.15 | 4.12 | 4.03 | 4.66 |
| Stiffness parameter $\beta$ after 5 weeks (right) | 3.38 | 4.00 | 2.72 | 4.04 | 4.31 |
| Change rate of stiffness parameter $\beta$ (right) | 67.2% | 96.6% | 66.1% | 100.4% | 92.4% |
| Initial stiffness parameter $\beta$ (left) | 5.27 | 4.46 | 3.72 | 4.38 | 5.01 |
| Stiffness parameter $\beta$ after 5 weeks (left) | 3.17 | 4.35 | 2.64 | 3.59 | 3.51 |
| Change rate of stiffness parameter $\beta$ (left) | 60.1% | 97.6% | 71.0% | 81.8% | 70.1% |

[Table 13]

Table 13 Tobaccos 16 to 20 (capsule 6)

| Comparative Example | 9-1 | 9-2 | 9-3 | 9-4 | 9-5 |
|---|---|---|---|---|---|
| Sex of subject | Male | Male | Male | Male | Male |
| Age of subject | 58 | 33 | 40 | 36 | 48 |
| Tobacco smoked | Tobacco 16 | Tobacco 17 | Tobacco 18 | Tobacco 19 | Tobacco 20 |
| Number of tobaccos smoked per day | 5 | 30 | 27 | 9 | 56 |
| Initial PWV (right) | 1534 | 1398 | 1399 | 1301 | 1533 |
| PWV after 5 weeks (right) | 1620 | 1423 | 1510 | 1244 | 1614 |
| Change rate of PWV (right) | 105.6% | 101.8% | 107.9% | 95.6% | 105.2% |
| Initial PWV (left) | 1558 | 1299 | 1399 | 1436 | 1444 |
| PWV after 5 weeks (left) | 1705 | 1374 | 1384 | 1176 | 1506 |
| Change rate of PWV (left) | 109.4% | 105.8% | 98.9% | 81.9% | 104.3% |
| Initial stiffness parameter $\beta$ (right) | 5.11 | 3.50 | 3.74 | 3.15 | 3.41 |
| Stiffness parameter $\beta$ after 5 weeks (right) | 4.64 | 3.89 | 4.43 | 3.03 | 3.79 |
| Change rate of stiffness parameter $\beta$ (right) | 90.8% | 111.2% | 118.4% | 96.1% | 111.1% |
| Initial stiffness parameter $\beta$ (left) | 5.09 | 3.20 | 3.81 | 3.61 | 3.26 |
| Stiffness parameter $\beta$ after 5 weeks (left) | 5.02 | 3.57 | 3.52 | 2.68 | 3.05 |
| Change rate of stiffness parameter $\beta$ (left) | 98.6% | 111.7% | 92.3% | 74.2% | 93.5% |

The means and standard deviations of change rates of PWVs and stiffness parameters β for the four subjects in Table 11 were as follows.

PWV (right): 96.2 ± 3.8%
PWV (left): 94.4 ± 1.8%
Stiffness parameter β (right): 89.5 ± 10.9%
Stiffness parameter β (left): 89.2 ± 1.5%

As shown in Table 11, seven of the eight measurements in total of the left and right PWVs of the four subjects decreased. Seven of the eight parameters in total of the left and right stiffness parameters β decreased. Thus, smoking the tobacco containing the capsule with 30% β-caryophyllene for 5 weeks resulted in improved PWVs and stiffness parameters β. From these results, β-caryophyllene was found to improve arterial stiffness and suppress increase in arterial stiffness.

The means and standard deviations of change rates of PWVs and stiffness parameters β for the five subjects in Table 12 were as follows.

PWV (right): 90.6 ± 5.2%
PWV (left): 87.2 ± 7.8%
Stiffness parameter β (right): 84.5 ± 16.6%
Stiffness parameter β (left): 76.1 ± 14.2%

As shown in Table 12, nine of the ten measurements in total of the left and right PWVs of the five subjects decreased. Nine of the ten parameters in total of the left and right stiffness parameters β decreased. Thus, smoking the tobacco containing the capsule with 15% β-caryophyllene for 5 weeks resulted in improved PWVs and stiffness parameters β. From these results, β-caryophyllene was found to improve arterial stiffness and suppress increase in arterial stiffness.

The means and standard deviations of change rates of PWVs and stiffness parameters β for the five subjects in Table 13 were as follows.

PWV (right): 103.2 ± 4.8%
PWV (left): 100.1 ± 10.8%
Stiffness parameter β (right): 105.5 ± 11.6%
Stiffness parameter β (left): 94.1 ± 13.5%

As shown in Table 13, only three of the ten measurements in total of the left and right PWVs of the five subjects decreased. Only six of the ten parameters in total of the left and right stiffness parameters β decreased. Thus, smoking the tobacco containing the placebo capsule without β-caryophyllene for 5 weeks did not result in notable improvement in PWVs and stiffness parameters β.

[Necessary amount of β-caryophyllene]

(1) As shown in Examples, vascular fibras were destroyed in 2 weeks if one mouse (body weight: 18 g) took 2 mg, i.e., 0.11 g/kg, of nicotine per day. In consideration of load placed on the mouse, this corresponds to the uptake of 7.7 g of nicotine per day in terms of humans because the average body weight of 40-year-old Japanese males is 70 kg. For example, provided that a nicotine intake per tobacco is 0.1 to 1 mg, the daily intake of nicotine is 2 to 20 mg by smoking one box (20 tobaccos) a day. Thus, the nicotine intakes by the mice of Example 5 and Comparative Example 8 are approximately 385 to 3850 times that in the case of smoking one box a day.
The mice of Example 5 and Comparative Example 7 involving the pulmonary uptake of caryophyllene were exposed to β-caryophyllene in the caryophyllene-filled flask for 1 hour each for 3 days among 7 days. This means exposure for 0.43 hours on average per day, and the inhalation intake of β-caryophyllene is 23 μg, i.e., 1.3 mg/kg. As described above, since the nicotine intakes by the mice are approximately 385 to 3850 times that in the case of smoking 20 tobaccos a day, the necessary amount of β-caryophyllene for smokers is approximately 1/3850 to 1/385 of the amount for the mice and is 468 ng/kg to 4680 ng/kg per day. Thus, the necessary amount of β-caryophyllene for a smoker having a body weight of 70 kg is 32.8 μg to 328 μg per day.
(2) From Example 3, the amount of β-caryophyllene contained in mainstream smoke when seamless capsules (approximately 20 μL each) filled with 100% by mass and 15% by mass of β-caryophyllene (capsule content liquid:

19.3 mg) were each inserted into a tobacco filter, then broken, and aspirated by lighting the tobacco was 2.99 mg and 0.29 mg, respectively.

Thus, 59.8 mg and 5.8 mg, respectively, of β-caryophyllene per day can be taken by smoking 20 tobaccos a day. This corresponds to 1.4 to 145 times the necessary amount of β-caryophyllene described above. Thus, it was found that provided that the concentration of β-caryophyllene is 15% by mass in approximately 20 μL of a seamless capsule, nicotine-induced vascular destruction ascribable to smoking can be prevented even for a tobacco having 1 mg of nicotine.

(3) According to Example 4, the serum concentration of β-caryophyllene was 8.8 ng/mL (8.8 ppb by mass) when β-caryophyllene was orally administered at 20 μg per g of body weight to the laboratory mice using a sonde.

The β-caryophyllene intake is approximately 6.7 times and the serum concentration is approximately 0.087 times, as compared with the mouse that underwent the pulmonary uptake of β-caryophyllene in Example 1. Therefore, for obtaining the serum concentration of β-caryophyllene by oral uptake at the same level as that for pulmonary uptake, the uptake of β-caryophyllene in an amount of approximately 77 times is necessary.

Thus, it was able to be assumed that the necessary amount of β-caryophyllene for a human who smoke 20 tobaccos a day is 450 mg by oral uptake, which can achieve the serum concentration of β-caryophyllene at the same level as that for the mouse that underwent pulmonary uptake in Example 1. This corresponds to 55 seamless capsules containing 15% by mass of β-caryophyllene.

(4) As shown in Example 1, when the same mouse as that used in the experiment was exposed to β-caryophyllene for 60 minutes, the amount of the β-caryophyllene brought into the body by inhalation was 54 μg (3.0 mg/kg). By contrast, the concentration of β-caryophyllene in serum from the mouse exposed to β-caryophyllene for 60 minutes in Example 4 was 34 ng/mL, the concentration of β-caryophyllene contained in thoracic aorta therefrom was 751 ng/g, and the concentration of β-caryophyllene contained in abdominal aorta therefrom was 403 ng/g. On the other hand, as described above, the amount of caryophyllene brought into a human who smokes, per day, 20 tobaccos provided with a capsule supplemented with 15% caryophyllene in a filter is 5.8 mg (0.083 mg/kg). Therefore, according to calculation from the bioavailability of the mouse described above, the putative concentration of β-caryophyllene in serum can be 0.93 ng/mL, the putative concentration of β-caryophyllene contained in thoracic aorta can be 21 ng/g, and the putative concentration of β-caryophyllene contained in abdominal aorta can be 11 ng/g. From the experiment to inhibit the degradation of extracellular matrix in an aortic tissue, the effect was confirmed at a caryophyllene concentration of 100 nmol/L (20 ng/mL) in an aortic vascular tissue. This suggested that a similar effect is also obtained for a human who smokes tobaccos containing a capsule supplemented with 15% caryophyllene.

[Change in concentration of β-caryophyllene in serum]

From the results of Examples, change in the concentration of β-caryophyllene in serum in the case of smoking 20 tobaccos a day at a frequency of one tobacco per hour was estimated by preparing an easy-to-disintegrate seamless capsule (content liquid: 19.3 mg) supplemented with 15% by mass of β-caryophyllene, and smoking a tobacco with a tobacco filter incorporating the seamless capsule after scatter of the content liquid by the disintegration of the capsule at the time of smoking.

Specifically, from the results of Example 1, the intake of β-caryophyllene by exposure for 60 minutes in the caryophyllene-filled flask was 54 μg (3.0 mg/kg), whereas the serum concentration of β-caryophyllene was 102 ng/mL (102 ppb by mass). From the results of the test using the capsule 2 in Example 3, the amount of β-caryophyllene brought into a human was 0.29 mg (0.41 μg/kg) per tobacco in the experiment using a smoking machine. Since the serum concentration of β-caryophyllene is considered to be proportionate to an intake per body weight, the serum concentration of β-caryophyllene after smoking of one tobacco is presumably 0.14 ng/mL (0.14 ppb by mass).

Provided that the half-life of the serum concentration of β-caryophyllene is 85.4 minutes, it was able to be presumed that the serum concentration varied as shown in Figure 2. In this case, the average serum concentration of β-caryophyllene per day was 0.24 ng/mL (0.24 ppb by mass).

## Claims

1. A composition for the treatment of vascular disease, for the prevention of vascular disease, for the treatment of hypertension or for the prevention of hypertension, comprising caryophyllene.

2. The composition according to claim 1, wherein the caryophyllene improves arterial stiffness or suppresses increase in arterial stiffness.

3. The composition according to claim 1, wherein the caryophyllene suppresses the degradation of vascular extracellular matrix.

4. The composition according to claim 1, wherein the caryophyllene suppresses the destruction of vascular fibra or repairs vascular fibra.

5. The composition according to claim 1, wherein the caryophyllene suppresses the destruction of vascular elastic lamina or repairs vascular elastic lamina.

6. The composition according to claim 1, wherein the caryophyllene inhibits matrix metalloprotease.

7. The composition according to claim 1, wherein the caryophyllene suppresses blood pressure elevation.

8. A composition for improving skin quality, comprising caryophyllene.

9. The composition according to any of claims 1 to 8, wherein the caryophyllene is obtained from clove, caraway, basil, oregano, hop, cinnamon, Ceylon cinnamon tree, rosemary, *Cannabis L.,* hemp, cannabis, black pepper, lavender, malabathrum, cananga tree, copaiba, melegueta pepper, curry leaf and/or an essential oil thereof.

10. The composition according to any of claims 1 to 9, wherein the caryophyllene is a chemically synthesized compound.

11. The composition according to any of claims 1 to 10, further comprising one or more members selected from the group consisting of a solvent and a fragrance.

12. The composition according to any of claims 1 to 11, wherein a dosage form is a capsule.

13. A tobacco filter comprising the composition according to any of claims 1 to 11.

14. A tobacco filter incorporating the capsule according to claim 12.

15. A tobacco comprising the tobacco filter according to claim 13 or 14.

16. A smoking tool comprising the tobacco filter according to claim 13 or 14.

17. A tobacco comprising the composition according to any of claims 1 to 11.

18. A food or drink comprising the composition according to any of claims 1 to 12.

19. A medicament comprising the composition according to any of claims 1 to 12.

20. The medicament according to claim 19, wherein the medicament is an oral composition or a pulmonary composition.

21. A method for treating vascular disease, a method for treating hypertension, a method for preventing vascular disease, or a method for preventing hypertension, comprising administering a composition comprising caryophyllene.

22. The method according to claim 21, wherein the caryophyllene improves arterial stiffness or suppresses increase in arterial stiffness to treat or prevent vascular disease or hypertension.

23. The method according to claim 21, wherein the caryophyllene suppresses the degradation of vascular extracellular matrix to treat or prevent vascular disease or hypertension.

24. The method according to claim 21, wherein the caryophyllene suppresses the destruction of vascular fibra or repairs vascular fibra to treat or prevent vascular disease or hypertension.

25. The method according to claim 21, wherein the caryophyllene suppresses the destruction of vascular elastic lamina or repairs vascular elastic lamina to treat or prevent vascular disease or hypertension.

26. The method according to claim 21, wherein the caryophyllene inhibits matrix metalloprotease to treat or prevent vascular disease or hypertension.

27. The method according to claim 21, wherein the caryophyllene suppresses blood pressure elevation to treat or prevent vascular disease or hypertension.

28. A method for improving skin quality, comprising administering a composition comprising caryophyllene.

29. The method according to any of claims 21 to 28, wherein the administration is pulmonary uptake or oral uptake.

30. The method according to claim 29, wherein the pulmonary uptake is inhalation mediated by a filter of an inhalation tool.

31. A composition comprising caryophyllene for use in the treatment of vascular disease, the treatment of hypertension, the prevention of vascular disease, or the prevention of hypertension.

[Figure 1]

Absorbent cotton with 10 mL of caryophyllene absorbed therein

Volatilization of β-caryophyllene

Inhalation of volatilized β-caryophyllene by mouse in flask

[Figure 2]

## INTERNATIONAL SEARCH REPORT

| | International application No. |
|---|---|
| | **PCT/JP2021/040234** |

**A. CLASSIFICATION OF SUBJECT MATTER**

*A61K 31/015*(2006.01)i; *A23L 33/10*(2016.01)i; *A61K 8/31*(2006.01)i; *A61P 9/10*(2006.01)i; *A61P 9/12*(2006.01)i; *A61P 9/14*(2006.01)i; *A61Q 19/00*(2006.01)i

FI: A61K31/015; A61P9/14; A61P9/12; A61P9/10; A61K8/31; A61Q19/00; A23L33/10

According to International Patent Classification (IPC) or to both national classification and IPC

**B. FIELDS SEARCHED**

Minimum documentation searched (classification system followed by classification symbols)

A61K31/015; A23L33/10; A61K8/31; A61P9/10; A61P9/12; A61P9/14; A61Q19/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | TIAN, X. et al. β-Caryophyllene Protects In Vitro Neurovascular Unit against Oxygen-Glucose Deprivation and Re-oxygenation-Induced Injury. Journal of Neurochemistry. 2016, 139(5), pp. 757-768 abstract, fig. 4, 7 | 1-7, 21-27, 31 |
| Y | | 9-20, 29-30 |
| X | GULLUNI, N. et al. Cannabis Essential Oil: A Preliminary Study for the Evaluation of the Brain Effects. Evidence-Based Complementary and Alternative Medicine. 2018, vol. 2018, Article ID 1709182, pp. 1-11 abstract, p. 3, right column, column 2.6., tables 1, 3 | 1-7, 21-27, 31 |
| Y | | 9-20, 29-30 |
| X | FUKUOKA, K. et al. Inhibitory Actions of Several Natural Products on Proliferation of Rat Vascular Smooth Muscle Cells Induced by Hsp60 from Chlamydia pneumoniae J138. Journal of Agricultural and Food Chemistry. 2004, 52(20), pp. 6326-6329 abstract, fig. 3, 5, p. 6327, left column, paragraph [0003] | 1-7, 21-27, 31 |
| Y | | 9-20, 29-30 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **24 November 2021** | **07 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

### INTERNATIONAL SEARCH REPORT

| International application No. |
| --- |
| **PCT/JP2021/040234** |

**C.    DOCUMENTS CONSIDERED TO BE RELEVANT**

| Category* | Citation of document, with indication, where appropriate, of the relevant passages | Relevant to claim No. |
| --- | --- | --- |
| Y | ADEFEGHA, S. A. et al. Essential Oil Composition, Antioxidant, Antidiabetic and Antihypertensive Properties of Two Afromomum Species. J.Oleo. Sci. 2017, 66(1), pp. 51-63 abstract | 9-20, 29-30 |
| Y | WO 2018/94359 A1 (BIOTECH INSTITUTE, LLC) 24 May 2018 (2018-05-24) examples 2, 3 | 11-20, 29-30 |
| Y | WO 2019/130500 A1 (JAPAN TOBACCO INC) 04 July 2019 (2019-07-04) paragraph [0002] | 11-20, 29-30 |
| Y | WO 2013/111281 A1 (JAPAN TOBACCO INC) 01 August 2013 (2013-08-01) claim 9, paragraph [0035] | 11-20, 29-30 |
| Y | JP 6603817 B1 (SUNSHO PHARMACEUTICAL CO., LTD.) 06 November 2019 (2019-11-06) paragraph [0007] | 11-20, 29-30 |
| X | JP 2013-184953 A (SHISEIDO CO LTD) 19 September 2013 (2013-09-19) claim 1, paragraphs [0002], [0015] | 8, 28 |
| A | IRRERA, N. et al. β-Caryophyllene Mitigates Collagen Antibody Induced Arthritis (CAIA) in Mice Through a Cross-Talk between CB2 and PPAR-γ Receptors. Biomolecules. 2019, 9, 326, pp. 1-15 entire text | 1-31 |
| P, X | CUNHA, R. M. D. et al. Vasorelaxant effect induced by the essential oil of Ocotea duckei vattimo leaves and its main constituent, trans-caryophyllene, in rat mesenteric artery. Journal of Applied Life Sciences International. December 2020, 23(12), pp. 31-39, JALSI.63877 entire text | 1-31 |

Form PCT/ISA/210 (second sheet) (January 2015)

| | International application No.<br>**PCT/JP2021/040234** |
|---|---|

| Patent document<br>cited in search report | Publication date<br>(day/month/year) | Patent family member(s) | Publication date<br>(day/month/year) |
|---|---|---|---|
| WO 2018/94359 A1 | 24 May 2018 | JP 2020-513416 A<br>US 2019/0365664 A1<br>EP 3541169 A1<br>KR 10-2019-0097038 A<br>CN 110225748 A | |
| WO 2019/130500 A1 | 04 July 2019 | US 2020/0281261 A1<br>paragraphs [0003]-[0004]<br>EP 3733001 A1<br>KR 10-2020-0090212 A<br>CN 111491525 A | |
| WO 2013/111281 A1 | 01 August 2013 | CN 104080898 A<br>KR 10-2014-0116531 A<br>HK 1198258 A<br>RU 2014134518 A<br>KR 10-2016-0114188 A | |
| JP 6603817 B1 | 06 November 2019 | EP 3711497 A1<br>paragraph [0008]<br>JP 2020-115847 A<br>WO 2020/148924 A1 | |
| JP 2013-184953 A | 19 September 2013 | WO 2013/132873 A1<br>TW 201340987 A | |

Form PCT/ISA/210 (patent family annex) (January 2015)

32

## REFERENCES CITED IN THE DESCRIPTION

**Patent documents cited in the description**

- JP 5047285 B
- JP 10506841 W
- JP 5581446 B

**Non-patent literature cited in the description**

- **SHUANGXI WANG ; CHENG ZHANG ; MIAO ZHANG ; BIN LIANG ; HUAIPING ZHU ; JIYEON LEE ; BENOIT VIOLLET ; LIJUN XIA ; YUN ZHANG ; MING-HUI ZOU.** *Nature Medicine,* 2012, vol. 18 (6), 902-912
- **HIRONA KUGO ; NOBUHIRO ZAIMA ; MEGUMI ONOZATO ; CHIE MIYAMOTO ; KEISUKE HASHIMOTO ; KENICHI YANAGIMOTO ; TATSUYA MORIYAMA.** *Food Funct.,* 2017, vol. 8, 2829-2835
- **HIRONA KUGO ; CHIE MIYAMOTO ; AYAKA SAWARAGI ; KIYOTO HOSHINO ; YUKA HAMATANI ; SHINICHI MATSUMURA ; YURI YOSHIOKA ; TATSUYA MORIYAMA ; NOBUHIRO ZAIMA.** *J. Oleo Sci.,* 2019, vol. 68 (1), 79-85
- **WANIDA SUKKETSIRI ; KIYOTO HOSHINO ; HIRONA KUGO ; TOMOMI NAKAMURA ; TSUKASA SASOH ; TATSUYA MORIYAMA ; NOBUHIRO ZAIMA.** *J. Oleo Sci.,* 2019, vol. 68, 1241-1249
- **KIYOTO HOSHINO ; HIRONA KUGO ; CHIE MIYAMOTO ; KEISUKE HASHIMOTO ; HIROSHI MURASE ; MASATOSHI MIZUNO ; TATSUYA MORIYAMA ; NOBUHIRO ZAIMA.** *J. Nutr. Sci. Vitaminol.,* 2020, vol. 66, 75-81
- **SHUJI SETOZAKI ; KENJI MINAKATA ; HIDETOSHI MASUMOTO ; SHINGO HIRAO ; KAZUHIRO YAMAZAKI ; KOICHIRO KUWAHARA ; TADASHI IKEDA ; RYUZO SAKATA.** *J. Vascular Surgery,* 2017, vol. 65, 180-1812
- Jikken Dobutsu Gaku Kakuron (Details of Experimental Zoology in English). Asakura Publishing Co., Ltd, 1972
- **SHIRAI, K. ; UTINO, J. ; OTSUKA, K. ; TAKATA, M.** *J. Atheroscler Thromb.,* 2006, vol. 13, 101-107